(19) Europäisches Patentamt
European Patent Office
Office européen des brevets

(11) Veröffentlichungsnummer: **0 451 654 B1**

# (12) EUROPÄISCHE PATENTSCHRIFT

(45) Veröffentlichungstag der Patentschrift: **07.09.94**

(51) Int. Cl.⁵: **C07D 211/90**, A61K 31/44

(21) Anmeldenummer: **91105115.9**

(22) Anmeldetag: **30.03.91**

Die Akte enthält technische Angaben, die nach dem Eingang der Anmeldung eingereicht wurden und die nicht in dieser Patentschrift enthalten sind.

## (54) Verwendung von N-alkylierten 1,4-Dihydropyridindicarbonsäureestern als Arzneimittel, neue Verbindungen und Verfahren zu Ihrer Herstellung.

(30) Priorität: **11.04.90 DE 4011695**

(43) Veröffentlichungstag der Anmeldung:
**16.10.91 Patentblatt 91/42**

(45) Bekanntmachung des Hinweises auf die Patenterteilung:
**07.09.94 Patentblatt 94/36**

(84) Benannte Vertragsstaaten:
**AT BE CH DE DK ES FR GB GR IT LI LU NL SE**

(56) Entgegenhaltungen:
**DE-A- 1 813 436**
**DE-A- 1 923 990**
**DE-A- 1 963 188**
**DE-A- 2 210 672**
**GB-A- 2 015 516**
**GB-A- 2 192 132**

(73) Patentinhaber: **BAYER AG**
**D-51368 Leverkusen (DE)**

(72) Erfinder: **Behner, Otto, Dr.**
**In den Birken 83**
**W-5600 Wuppertal 1 (DE)**
Erfinder: **Wollweber, Hartmut, Dr.**
**In den Birken 73**
**W-5600 Wuppertal 1 (DE)**
Erfinder: **Rosen, Bruno, Dr.**
**Marienburger Strasse 65**
**W-5603 Wülfrath (DE)**
Erfinder: **Zaiss, Siegfried, Dr.**
**Farnweg 3**
**W-5600 Wuppertal 1 (DE)**
Erfinder: **Goldmann, Siegfried, Dr.**
**Am Osterholz 91**
**W-5600 Wuppertal 11 (DE)**

**EP 0 451 654 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung von teilweise bekannten N-alkylierten 1,4-Dihydropyridindicarbonsäureestern als hämorheologisches Arzneimittel, neue Wirkstoffe und Verfahren zu ihrer Herstellung, insbesondere ihre Verwendung als Arzneimittel bei akuten und chronisch ischämischen Erkrankungen, die mit Mikrozirkulationsstörungen verbunden sind. Diese Wirkung kann sowohl im peripheren als auch im cerebralen Gefäßsystem auftreten.

Es ist bekannt, daß 1,4-Dihydropyridindicarbonsäureester eine calciumantagonistische oder calciumagonistische Wirkung besitzen, und somit als kreislaufbeeinflussende Mittel eingesetzt werden können [vgl. DOS 25 06 987; DE 22 10 667].

In der EP 240 828 werden blutdrucksenkende 1,4-Dihydropyridine mit hämorheologischen Eigenschaften beschrieben.

Auch in der DE 37 20 509 wird die Verwendung heterocyclisch substituierter, blutdrucksenkender 1,4-Dihydropyridine als hämorheologische Mittel publiziert.

Es wurde nun gefunden, daß die teilweise bekannten und neuen N-alkylierten 1,4-Dihydropyridindicarbonsäureester der allgemeinen Formel (I)

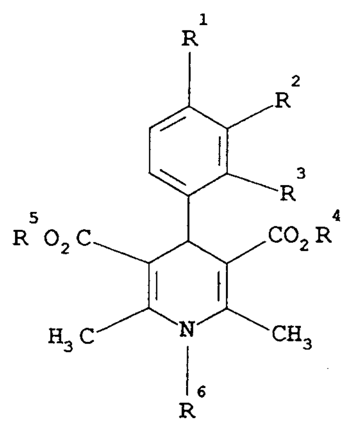

(I)

in welcher

- $R^1$ für Wasserstoff, Nitro, Trifluormethyl, Trifluormethoxy, Cyano, Fluor, Chlor, Brom oder Methyl steht,
- $R^2$ für Wasserstoff, Fluor, Chlor, Brom, Nitro, Hydroxy, Trifluormethyl oder Methyl steht,
- $R^3$ für Wasserstoff oder Cyano steht, oder
- $R^2$ und $R^3$ gemeinsam einen ankondensierten Benzoring bilden,
- $R^4$ und $R^5$ gleich oder verschieden sind und für geradkettiges oder verzweigtes Alkyl mit bis zu 6 Kohlenstoffatomen stehen, das gegebenenfalls durch Methoxy substituiert ist, und
- $R^6$ für geradkettiges oder verzweigtes Alkyl mit bis zu 8 Kohlenstoffatomen oder für Cyclopropyl steht,

überraschenderweise bei blutdruckneutralem Verhalten eine starke hämorheologische Wirkung besitzen und die Durchblutung, insbesondere die Mikrozirkulation verbessern und somit geeignet sind zur Verwendung bei der Bekämpfung akuter und chronisch ischämischer Erkrankungen.

Besonders bevorzugt sind Verbindungen der allgemeinen Formel (I)

in welcher

- $R^1$ für Wasserstoff, Nitro, Trifluormethyl, Trifluormethoxy, Cyano, Fluor, Brom, Chlor oder Methyl steht,
- $R^2$ für Wasserstoff, Fluor, Chlor, Nitro, Hydroxy, Trifluormethyl oder Methyl steht,
- $R^3$ für Wasserstoff oder Cyano steht,

oder

- $R^2$ und $R^3$ gemeinsam einen ankondensierten Benzoring bilden,
- $R^4$ und $R^5$ gleich oder verschieden sind und für geradkettiges oder verzweigtes Alkyl mit bis zu 6 Kohlenstoffatomen stehen, das gegebenenfalls durch Methoxy substituiert ist

und

- $R^6$ für Methyl, Ethyl oder Cyclopropyl steht,

bei der Bekämpfung von akuten und chronisch ischämischen Erkrankungen.

Die erfindungsgemäßen Verbindungen zeigen ein nicht vorhersehbares, wertvolles pharmakologisches Wirkspektrum.

Verbunden mit einem blutdruckneutralen Verhalten in einem Dosis-Bereich bis mindestens 10 mg/kg i.v. und 30 mg/kg p.o. steigern sie die Durchblutung, insbesondere die Mikrozirkulation durch Beeinflussung der Verformbarkeit der Erythozyten sowie der Inhibition der Aktivierung und Adhäsion der Leukozyten.

Die Blutdruckneutralität wird an folgenden, für Dihydropyridine typischen Modellen, ermittelt: In SH-Ratten nach p.o. Gabe durch Messung an der Schwanzarterie (Riva Rocci Methode) und an narkotisierten Wistar-Ratten nach i.v. Gabe (blutig über Katheter in A. carotis). Als blutdruckneutral werden solche Verbindungen bezeichnet, die in beiden Testmodellen mit der angegebenen Dosis den Blutdruck bis zu maximal 20 % vom Ausgangswert senken. Der Abstand der therapeutischen Dosis und der einsetzenden Blutdruckwirkung ist mindestens Faktor 10, in der Regel Faktor ≧ 30, insbesondere ≧ 100.

Sie können deshalb für die Herstellung von Arzneimitteln zur Behandlung von akuten und chronisch ischämischen Erkrankungen, wie Claudicatio intermittens, Myokardinfarkt, Gehirninfarkt sowie von Reperfusionsschäden und Schock eingesetzt werden.

Die Erfindung betrifft außerdem neue 1,4-Dihydropyridindicarbonsäureester, die im folgenden aufgeführt werden:

1,2,6-Trimethyl-4-(1-naphthyl)-1,4-dihydro-pyridin-3,5-dicarbonsäuredibutylester
1,2,6-Trimethyl-4-(4-fluorphenyl)-1,4-dihydro-pyridin-3,5-dicarbonsäurediethylester
1,2,6-Trimethyl-4-(2-cyanphenyl)-1,4-dihydro-pyridin-3,5-dicarbonsäuredipropylester
1,2,6-Trimethyl-4-(4-nitrophenyl)-1,4-dihydro-pyridin-3,5-dicarbonsäuredibutylester
1,2,6-Trimethyl-4-(4-trifluormethylphenyl)-1,4-dihydropyridin-3,5-dicarbonsäuredimethylester
1,2,6-Trimethyl-4-(4-trifluormethylphenyl)-1,4-dihydropyridin-3,5-dicarbonsäure-methyl-propylester
1,2,6-Trimethyl-4-(4-trifluormethylphenyl)-1,4-dihydropyridin-3,5-dicarbonsäure-methyl-isopropylester
1,2,6-Trimethyl-4-(4-trifluormethylphenyl)-1,4-dihydropyridin-3,5-dicarbonsäure-methyl-(1,2-dimethylpropyl)-ester
1,2,6-Trimethyl-4-(4-trifluormethylphenyl)-1,4-dihydropyridin-3,5-dicarbonsäure-methyl-(2-methoxyethyl)ester
1,2,6-Trimethyl-4-(3-fluorphenyl)-1,4-dihydro-pyridin-3,5-dicarbonsäuredimethylester
1,2,6-Trimethyl-4-(3-methylphenyl)-1,4-dihydro-pyridin-3,5-dicarbonsäuredipropylester
1,2,6-Trimethyl-4-(4-bromphenyl)-1,4-dihydro-pyridin-3,5-dicarbonsäuredimethylester
1,2,6-Trimethyl-4-(4-bromphenyl)-1,4-dihydro-pyridin-3,5-dicarbonsäuredipropylester
1,2,6-Trimethyl-4-(4-bromphenyl)-1,4-dihydro-pyridin-3,5-dicarbonsäuredibutylester
1,2,6-Trimethyl-4-(4-cyanphenyl)-1,4-dihydro-pyridin-3,5-dicarbonsäuredipropylester
1-Cyclopropyl-2,6-dimethyl-4-(3-trifluormethylphenyl)-1,4-dihydro-pyridin-3,5-dicarbonsäurediethylester
1-Ethyl-2,6-dimethyl-4-(4-trifluormethylphenyl)-1,4-dihydro-pyridin-3,5-dicarbonsäuredimethylester
1-Cyclopropyl-2,6-dimethyl-4-(4-trifluormethylphenyl)-1,4-dihydro-pyridin-3,5-dicarbonsäuredimethylester
1-Cyclopropyl-2,6-dimethyl-4-(4-trifluormethylphenyl)-1,4-dihydro-pyridin-3,5-dicarbonsäurediethylester
1,2,6-Trimethyl-4-(4-trifluormethylphenyl)-1,4-dihydropyridin-3,5-dicarbonsäurediisopropylester
1,2,6-Trimethyl-4-(4-methyl-3-nitrophenyl))-1,4-dihydropyridin-3,5-dicarbonsäuredimethylester
1,2,6-Trimethyl-4-(4-methyl-3-nitrophenyl)-1,4-dihydropyridin-3,5-dicarbonsäuredipropylester
1,2,6-Trimethyl-4-(4-methyl-3-nitrophenyl)-1,4-dihydropyridin-3,5-dicarbonsäuredibutylester
1,2,6-Trimethyl-4-(4-methyl-3-nitrophenyl)-1,4-dihydropyridin-3,5-dicarbonsäurediethylester
1,2,6-Trimethyl-4-(3-chlor-4-nitrophenyl)-1,4-dihydropyridin-3,5-dicarbonsäurediethylester
1,2,6-Trimethyl-4-(4-chlor-3-trifluormethylphenyl)-1,4-dihydro-pyridin-3,5-dicarbonsäuredimethylester
1,2,6-Trimethyl-4-(4-methyl-3-trifluormethylphenyl)-1,4-dihydro-pyridin-3,5-dicarbonsäuredimethylester
1,2,6-Trimethyl-4-(3-hydroxy-4-nitrophenyl)-1,4-dihydropyridin-3,5-dicarbonsäuredimethylester
1-Cyclopropyl-2,6-dimethyl-4-(4-trifluormethoxyphenyl)-1,4-dihydro-pyridin-3,5-dicarbonsäuredimethylester
1-Cyclopropyl-2,6-dimethyl-4-(4-trifluormethoxyphenyl)-1,4-dihydro-pyridin-3,5-dicarbonsäurediethylester
1,2,6-Trimethyl-4-(4-trifluormethylphenyl)-1,4-dihydropyridin-3,5-dicarbonsäure-isopropyl-(2-methoxyethyl)-ester
1,2,6-Trimethyl-4-(4-trifluormethylphenyl)-1,4-dihydropyridin-3,5-dicarbonsäurediethylester
1-Cyclopropyl-2,6-dimethyl-4-(4-trifluormethylphenyl)-1,4-dihydro-pyridin-3,5-dicarbonsäure-methyl-ethylester
1-Cyclopropyl-2,6-dimethyl-4-(4-trifluormethylphenyl)-1,4-dihydro-pyridin-3,5-dicarbonsäure-propyl-(2-methoxyethyl)ester
1-Cyclopropyl-2,6-dimethyl-4-(4-trifluormethylphenyl)-3,5-dicarbonsäure-1,4-dihydro-pyridin-isopropyl-(2-methoxyethyl)-ester
1-Cyclopropyl-2,6-dimethyl-4-(4-fluorphenyl)-1,4-dihydro-pyridin-3,5-dicarbonsäurediethylester
1-Cyclopropyl-2,6-dimethyl-4-(4-fluorphenyl)-1,4-dihydro-pyridin-3,5-dicarbonsäuredimethylester

1-Cyclopropyl-2,6-dimethyl-4-(4-trifluorphenyl)-1,4-dihydro-pyridin-3,5-dicarbonsäure-propyl-butylester
1-Cyclopropyl-2,6-dimethyl-4-(4-trifluormethylphenyl)-1,4-dihydro-pyridin-3,5-dicarbonsäure-butyl-methylester
1-Cyclopropyl-2,6-dimethyl-4-(4-trifluormethylphenyl)-1,4-dihydro-pyridin-3,5-dicarbonsäure-ethyl-propylester
1-Cyclopropyl-2,6-dimethyl-4-(4-trifluormethylphenyl)-1,4-dihydro-pyridin-3,5-dicarbonsäure-butyl-ethylester
1-Cyclopropyl-2,6-dimethyl-4-(4-trifluormethylphenyl)-1,4-dihydro-pyridin-3,5-dicarbonsäure-ethyl-isopropylester.

Besonders bevorzugte Verbindungen sind die 1,4-Dihydropyridindicarbonsäureester, deren Phenylring in para-Position einfach durch Fluor, Brom oder durch die $CF_3$-Gruppe substituiert ist.

Ganz besonders bevorzugt sind folgende Verbindungen:
1,2,6-Trimethyl-4-(4-bromphenyl)-1,4-dihydro-pyridin-3,5-dicarbonsäuredimethylester (Ex 13)
1-Cyclopropyl-2,6-dimethyl-4-(4-trifluormethylphenyl)-1,4-dihydro-pyridin-3,5-dicarbonsäurediethylester (Ex 19)
1-Cyclopropyl-2,6-dimethyl-4-(4-trifluormethylphenyl)-1,4-dihydro-pyridin-3,5-dicarbonsäuredimethylester (Ex 18)
1,2,6-Trimethyl-4-(4-trifluormethylphenyl)-1,4-dihydro-pyridin-3,5-dicarbonsäuredimethylester (Ex 3)
1,2,6-Trimethyl-4-(4-trifluormethylphenyl)-1,4-dihydro-pyridin-3,5-dicarbonsäure-methyl-(1,2-dimethylpropyl)-ester.

Einige der erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Die erfindungsgemäßen Verbindungen der allgemeinen Formel (I) und die neuen Verbindungen können hergestellt werden, indem man

[A] Benzylidenverbindungen der allgemeinen Formel (II)

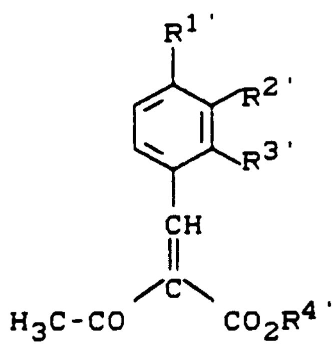

(II)

in welcher

$R^{1'}$, $R^{2'}$, $R^{3'}$ und $R^{4'}$ die oben angegebene Bedeutung von $R^1$, $R^2$, $R^3$ und $R^4$ haben und außerdem den jeweilen Bedeutungsumfang der oben aufgeführten neuen Verbindungen erfassen,

entweder

zunächst mit $\beta$-Aminocrotonsäureestern der allgemeinen Formel (III)

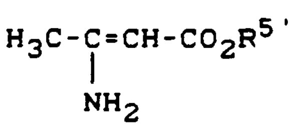

(III)

in welcher

$R^{5'}$ die oben angegebene Bedeutung von $R^5$ hat und außerdem den Bedeutungsumfang der oben aufgeführten neuen Verbindungen erfaßt,

in inerten Lösemitteln umsetzt und in einem letzten Schritt nach üblicher Methode die -NH-Funktion alkyliert,
oder
direkt die Verbindungen der allgemeinen Formel (II) gegebenenfalls in Anwesenheit von Lewissäuren wie Titantetrachlorid mit Verbindungen der allgemeinen Formel (IIIa)

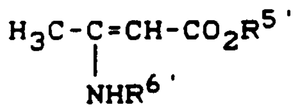

(IIIa)

in welcher
$R^{5'}$ die oben angegebene Bedeutung hat,
$R^{6'}$ die oben angegebene Bedeutung von $R^6$ hat und außerdem den Bedeutungsumfang der oben aufgeführten neuen Verbindungen erfaßt,

umsetzt,
oder
[B] Aldehyde der allgemeinen Formel (IV)

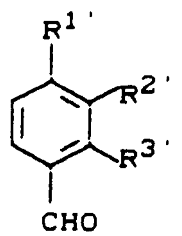

(IV)

in welcher
$R^{1'}$, $R^{2'}$ und $R^{3'}$ die unter Verfahren [A] angegebene Bedeutung haben,
zunächst mit β-Ketocarbonsäureestern der allgemeinen Formeln (V) und (Va)

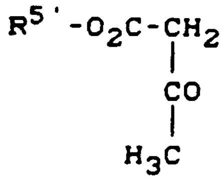

(V) und

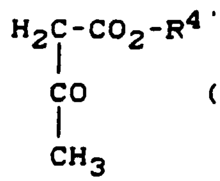

(Va)

in welcher
$R^{4'}$ und $R^{5'}$ ebenfalls die unter Verfahren [A] angegebene Bedeutung haben
umsetzt
und anschließend
entweder direkt mit Aminen oder den entsprechenden Aminhydrochloriden der allgemeinen Formel (VI)

$H_2N\text{-}R^{6'}$ (VI)

in welcher
$R^{6'}$ die oben unter Verfahren [A] angegebene Bedeutung hat,
umsetzt oder
zunächt mit Ammoniak in organischen, gegebenenfalls inerten, Lösemitteln nach üblicher Methode ringschließt und in einem letzten Schritt nach der oben aufgeführten Methode alkyliert,
und im Fall der enantiomerenreinen Ester zunächst die enantiomerenreinen Carbonsäuren herstellt und diese, gegebenenfalls über ein reaktives Säurederivat mit den entsprechenden Alkoholen nach üblicher

Methode verestert.

Die erfindungemäßen Verfahren zur Herstellung der neuen Verbindungen können durch folgendes Formelschema beispielhaft erläutert werden:

[A]

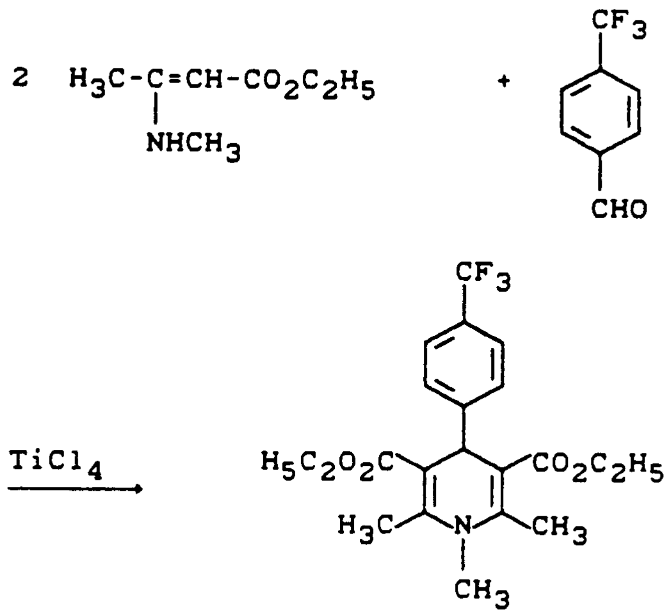


[B]

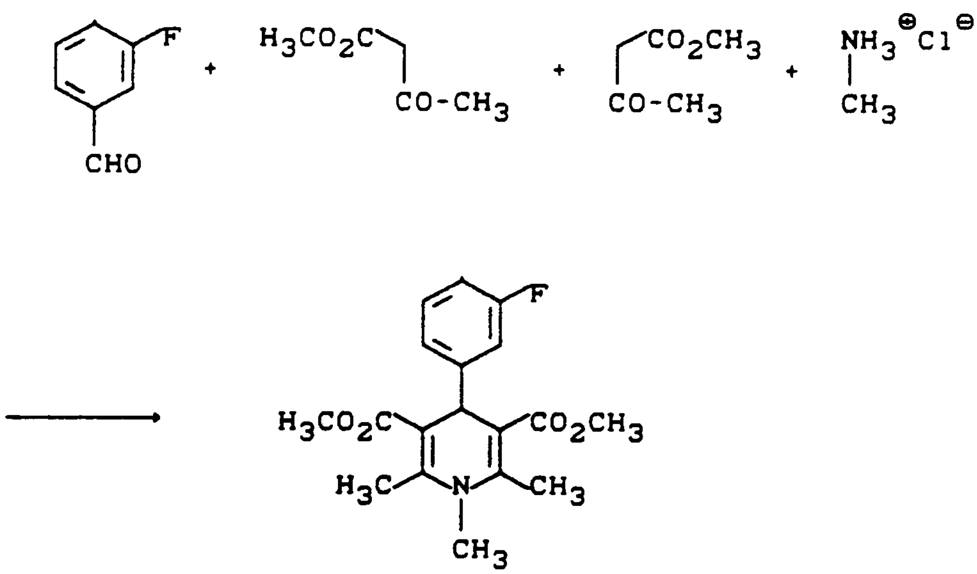

Verfahrensbeschreibung

Als Lösemittel kommen Wasser, oder organische Lösemittel in Frage, die sich unter den Reaktionsbedingungen nicht verändern. Hierzu gehören vorzugsweise Alkohole wie Methanol, Ethanol, Propanol, Isopropanol, Ether wie Diethylether, Dioxan, Tetrahydrofuran, Glykolmonomethylether oder Glykoldimethylether, oder Amide wie Dimethylformamid, Dimethylacetamid oder Hexamethylphosphorsäuretriamid, oder Eisessig, Dimethylsulfoxid, Acetonitril oder Pyridin.

Die Reaktionstemperaturen können in einem größeren Bereich variiert werden. Im allgemeinen arbeitet man zwischen +10°C und +150°C, vorzugsweise zwischen +20°C und +100°C, insbesondere bei der Siedetemperatur des jeweiligen Lösemittels.

Die Umsetzung kann bei Normaldruck, aber auch bei erhöhtem oder erniedrigtem Druck durchgeführt werden. Im allgemeinen arbeitet man bei Normaldruck.

Bei der Durchführung der erfindungsgemäßen Verfahrensvarianten A und B ist das Verhältnis der an der Reaktion beteiligten Stoffe beliebig. Im allgemeinen arbeitet man jedoch mit molaren Mengen der Reaktanden. Die Isolierung und Reinigung der erfindungsgemäßen Substanzen erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkristallisiert. In einigen Fällen kann es erforderlich sein, die erfindungsgemäßen Verbindungen durch Chromatographie zu reinigen.

Die Ylidenverbindungen der allgemeinen Formel (II) sind teilweise bekannt oder können nach bekannten Methoden hergestellt werden [vgl. H. Dornow und W. Sassenberg, Liebigs Ann. Chem. 602, 14 (1957)].

Die als Ausgangsstoffe eingesetzten Aldehyde der allgemeinen Formel (IV) sind bekannt oder können nach bekannten Methoden hergestellt werden [DOS 21 65 260; 24 01 665; T.D. Harris, G.P. Roth, J. Org. Chem. 44, 2004 (1979); W.J. Dale, H.E. Hennis, J. Am. Chem. Soc. 78, 2543 (1956); Chem. Abstr. 59, 13929 (1963)].

Die als Ausgangsstoffe eingesetzten $\beta$-Ketocarbonsäureester der allgemeinen Formel (V) und (Va) sind bekannt oder können nach bekannten Methoden hergestellt werden [D. Borrmann in Houben Weyl's "Methoden der organischen Chemie" Bd. VII/4, 230 (1968); Y. Oikawa, K. Sugano, O. Yonemitsu, J. Org. Chem. 43, 2087 (1978)].

Die als Ausgangsstoffe eingesetzten $\beta$-Aminocrotonsäureester der allgemeinen Formeln (III) und (IIIa) sind bekannt oder können nach bekannten Methoden hergestellt werden [DOS 2 228 377; F.A. Glickman, A.C. Cope, J. Am. Chem. Soc. 67, 1017 (1945)].

Die Verbindungen der allgemeinen Formel (VI) sind ebenfalls bekannt.

Als reaktive Säurederivate seien beispielsweise genannt: aktivierte Ester, Hydroxysuccinimidester, Säureimidazolide, Säurehalogenide, gemischte Anhydride oder die Umsetzung in Anwesenheit von Cyclohexylcarbodiimid.

Als Alkylierungsmittel bei dem Verfahren können beispielsweise ($C_1$-$C_8$)-Alkylhalogenide, Sulfonsäureester oder substituierte oder unsubstituierte ($C_1$-$C_6$)-Dialkylsulfate, vorzugsweise Methyliodid, p-Toluolsulfonsäureester oder Dimethylsulfat eingesetzt werden.

Die Alkylierung wird in den oben aufgeführten Lösemitteln bei Temperaturen von 0°C bis +150°C, vorzugsweise bei Raumtemperatur bis +100°C bei Normaldruck durchgeführt.

Als aktivierende Reagenzien zur Herstellung der reaktiven Säurederivate seien neben den anorganischen Halogeniden wie Thionylchlorid, Phosphortrichlorid oder Phosphorpentachlorid, oder dem Carbonyldiimidazol, Carbodiimide wie Cyclohexylcarbodiimid oder 1-Cyclohexyl-3-[2-(N-methyl-morpholino)ethyl]-carbodiimid-p-toluolsulfonat oder N-Hydroxyphthalimid oder N-Hydroxybenztriazol in Gegenwart von Dicyclohexylcarbodiimid beispielhaft erwähnt.

Als Lösungsmittel für die Umsetzung mit den entsprechenden Alkoholen eignen sich die oben aufgeführten Lösemittel mit Ausnahme der Alkohole.

Die Trennung der Diastereomerenpaare erfolgt nach bekannten Methoden wie Säulenchromatographie, fraktionierte Kristallisation oder Craigverteilung [zur Craigverteilung siehe beispielsweise "Verteilungsverfahren im Laboratorium", E. Hecher, Verlag Chemie GmbH, Weinheim, Bergstr. (1955)].

Die neuen und die bekannten erfindunsgemäßen Verbindungen zeigen ein nicht vorhersehbares, wertvolles pharmakologisches Wirkspektrum.

Die folgenden in vitro und in vivo Tests zeigen die interessanten Wirkungen der erfindungsgemäßen Verbindungen.

I) Erythrozytenfunktion

Die Verformbarkeit von Erythrozyten spielt für die Entstehung und den Verlauf akuter oder chronischer ischämischer Erkrankungen eine wesentliche Rolle. Sie bestimmt die Viskosität des Bluts und damit seine Verteilung in der Mikrozirkulation. Die angewendeten Tests erfassen verschiedene Determinanten:

Test a) mißt die Calciumpermeabilität ($^{45}$ Ca) nach Blockade der ATPasen durch Na-ortho-Vanadat. Dadurch kann Calcium in den Erythrozyten akkumulieren. Eine Konsequenz ist eine verminderte Flexibilität. Für Test a) sind $ED_{50}$-Werte (mol/l) für die Inhibition des Calciumeinstroms angegeben.

Test b) erfaßt die antihämolytische Wirkung der Substanzen ($ED_{50}$, mol/l). Hierbei werden calciumbeladene Erythrozyten unter hohen Schubspannungen durch kleine Poren gepreßt, sodaß Hämoglobin als

Ausdruck ihrer Hämolyse freigesetzt und gemessen wird. Die Verminderung der Hämoglobinfreisetzung ist die Meßgröße.
Test c) erfaßt die Filtrierbarkeit von calciumbeladenen Erythrozyten durch 5 µm große Poren ($ED_{50}$, mol/l). Hierbei spielt die Membranflexibilität unter geringem Kraftgradienten eine Rolle.
Test d) erfaßt die Viskosität von Erythrozytensuspensionen in Glaskapillaren (25 µm Durchmesser) bei niedrigen, in Gefäßgebieten hinter einer Stenose auftretenden Schubspannungen. Durch Erhöhung des extrazellulären Calciums steigt die Viskosität an.
Die Tabelle gibt die prozentuale Verbesserung der Viskosität bezogen auf Schädigung = 100% bei einer Testdosis von 10 ng/ml an.

a) Calciumpermeabilität von Erythrozyten:

Nach Blockade der membranständigen ATPasen durch Naortho-Vanadat (0,75 mM) wird die Calciumpermeabilität gemessen ($^{45}$ Ca-Methode). Akkumulation von Calcium vermindert die Flexibilität der Erythrozyten.

**Tabelle I:**

| Beispiel-Nr. | $ED_{50}$ der Inhibition (mol/l) |
|---|---|
| 3 | $5 \times 10^{-6}$ |
| 13 | $5 \times 10^{-6}$ |
| 21 | $5 \times 10^{-6}$ |
| 27 | $10^{-6}$ |

b) Antihämolytische Wirkung an Erythrozyten

Normale Erythrozyten werden unter hohen Schubspannungen hämolytisch. Besonders ausgeprägt ist die Hämolyse von calciumbeladenen Zellen. Dieses Maß für mechanische Stabilität wird zur Substanzcharakterisierung herangezogen. Meßgröße ist die Konzentration freien Hämoglobins im Medium.

Tabelle II:

| Beispiel-Nr. | $ED_{50}$ der antihämolytischen Wirkung (mol/l) |
|---|---|
| 2 | $10^{-7}$ |
| 13 | $5 \times 10^{-7}$ |
| 15 | $5 \times 10^{-6}$ |
| 21 | $5 \times 10^{-7}$ |
| 22 | $5 \times 10^{-7}$ |
| 26 | $3 \times 10^{-8}$ |
| 27 | $5 \times 10^{-8}$ |

c) Filtration von Erythrozyten

Filtration von Erythrozyten durch 5 µm große Siebporen ist ein etabliertes Verfahren zur Bestimmung der Verformbarkeit von Erythrozyten. Die Zellen werden in normalem Puffer für 30 min geschert, sodaß intrazellulär die Calciumkonzentration ansteigt und die Flexibilität vermindert wird.

Tabelle III:

| Bsp.-Nr. | $ED_{50}$ Verbesserung der Flexibilität gegen geschädigte Kontrolle (mol/l) |
|---|---|
| 1 | $5 \times 10^{-6}$ |
| 3 | $5 \times 10^{-6}$ |
| 26 | $5 \times 10^{-6}$ |
| 28 | $5 \times 10^{-6}$ |

d) Viskosität in Glaskapillaren

Die biophysikalisch, für die Durchblutung relevanten Wechselwirkungen von Erythrozyten kann in Glaskapillaren (Durchmesser 20-30 µm) untersucht werden. Die resultierende Viskosität hängt von dem Zustand der Zellen ab. Bei Calciumbeladung steigt die Viskosität an. Angegeben ist die prozentuale Verbesserung der Viskosität bezogen auf geschädigte, jedoch unbehandelte Kontrolle bei 0,7 Pa. Die Testdosis beträgt $10^{-8}$ g/ml.

Tabelle IV:

| Beispiel-Nr. | Effekt (%) |
|---|---|
| 3 | 143 |
| 13 | 120 |
| 14 | 206 |
| 15 | 75 |
| 17 | 62 |
| 18 | 208 |
| 24 | 226 |

II) Leukozytenfunktion

Im Modell der Hamsterbackentasche kann die Mikrozirkulation direkt beobachtet werden. Meßgrößen sind die Leukozytenadhäsion sowie Gefäßdurchmesser und Erythrozytengeschwindigkeit. Die Adhäsion wurde unter ischämischen und nicht ischämischen Versuchsbedingungen quantifiziert. Unter nicht ischämischen Bedingungen ist die Adhäsion im Bereich kleiner Venolen quantifiziert, unter ischämischen Bedingungen (10 min Durchblutungsstop) in kleinen Arteriolen. Die Ergebnisse der Kontrollversuche sind auf 100 % angegeben. Als Testdosis werden jeweils 0,1 mg/kg i.v. gewählt, die Ergebnisse sind Abnahme in % der Kontrolle.

Tabelle V:

| Beispiel-Nr. | nicht-ischämisch Kontrolle = 100 % | ischämisch Kontrolle = 100 % |
|---|---|---|
| 3 | 63 % | 31 % |
| 6 | 50 % | 56 % |
| 9 | 70 % | 36 % |
| 18 | 54 % | 32 % |
| 19 | 61 % | 34 % |

III) Blutdruck

Der klinische Kenntnisstand zeigt, daß antiischämische Wirkungen von Dihydropyridinen häufig durch eine Vasodilatation überdeckt werden. Es war daher Ziel, blutdruckunwirksame (d.h. Abstand hämorheologische Wirkung und blutdrucksenkende Wirkung ≧ 10) DHP's zu finden. Die folgende Tabelle zeigt die Dosen, bei denen bei p.o.-Gabe (SH-Ratte) bzw. i.v.-Gabe (narkotisierte Wistaratte) eine Blutdrucksenkung eintritt.

**Tabelle VI:**

| Beispiel-Nr. | p.o. (mg/kg) | i.v. (mg/kg) |
|---|---|---|
| 3 | > 30 | > 10 |
| 6 | > 30 | > 10 |
| 9 | > 30 | > 10 |
| 18 | > 30 | > 10 |
| 19 | > 100 | > 10 |

Die Tabelle zeigt, daß im Vergleich mit Modell II der Abstand der therapeutischen Wirkung und Blutdruckwirkung (i.v.) mindestens 100 beträgt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellungsbeispiele

Beispiel 1

1,2,6-Trimethyl-4-(4-fluorphenyl)-1,4-dihydro-pyridin-3,5-dicarbonsäurediethylester

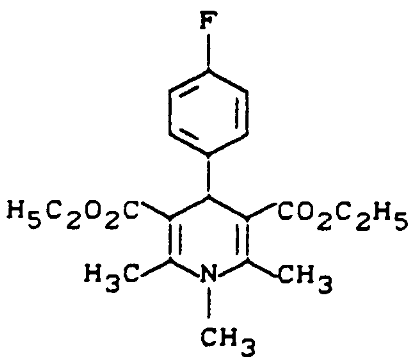

2,78 g (0,008 mol) 2,6-Dimethyl-4-(4-fluorphenyl)-1,4-dihydro-pyridin-3,5-dicarbonsäure-diethylester werden in 25 ml 1,2-Dimethoxyethan gelöst, mit 0,30 g (0,01 mol) 80%igem Natriumhydrid und nach 30 min mit 1,43 g (0,01 mol) Methyliodid versetzt. Man rührt 3 Stunden bei Raumtemperatur nach, neutralisiert mit verdünnter Salzsäure und dampft im Vakuum ein. Der Rückstand wird durch Chromatographie über Kieselgel (Methylenchlorid) gereinigt.
Ausbeute: 1,85 g (63,9% der Theorie).
Schmelzpunkt: 90 - 92 °C.

Beispiel 2

4-(3-Fluorphenyl)-1,2,6-trimethyl-1,4-dihydro-pyridin-3,5-dicarbonsäuredimethylester

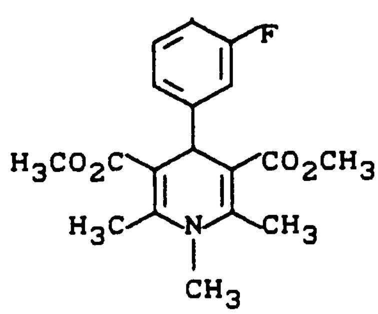

Eine Mischung aus 3,84 g (0,03 mol) 3-Fluorbenzaldehyd, 7,04 g (0,06 mol) Acetessigsäuremethylester und 2,07 g (0,03 mol) Methylamin-Hydrochlorid in 20 ml Pyridin wird 5 Stunden unter Rückfluß gerührt. Nach dem Abdestillieren des Pyridins wird zwischen Wasser und Methylenchlorid verteilt, die organische Phase gewaschen mit Wasser, getrocknet über Natriumsulfat und eingedampft. Der Rückstand wird aus Methanol umkristallisiert.
Schmp.: 117-118 °C
Ausbeute: 6,14 g (61,4% der Theorie)

Beispiel 3

1,2,6-Trimethyl-4-(4-trifluormethylphenyl)-1,4-dihydro-pyridin-3,5-dicarbonsäuredimethylester

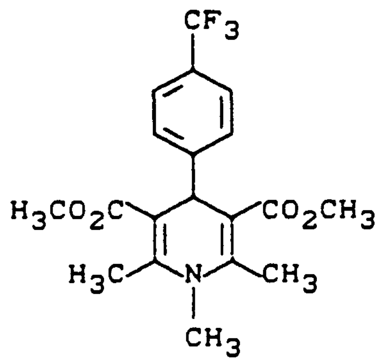

Eine Mischung aus 5,22 g (0,03 mol) 4-Trifluormethylbenzaldehyd, 7,04 g (0,06 mol) Acetessigsäuremethylester und 2,07 g (0,03 mol) Methylamin-Hydrochlorid in 20 ml Pyridin wird 5 Stunden unter Rückfluß gerührt. Nach dem Abdestillieren des Pyridins wird zwischen Wasser und Methylenchlorid verteilt, die organische Phase gewaschen mit Wasser, getrocknet über Natriumsulfat und eingedampft. Der Rückstand wird aus Methanol umkristallisiert.
Schmp.: 154 - 155 °C
Ausbeute: 7,88 g (68,5% der Theorie)
Die in den Tabellen 1 und 2 aufgeführten Beispiele wurden in Analogie zur Vorschrift des Beispiels 3 hergestellt.

**Tabelle 1:**

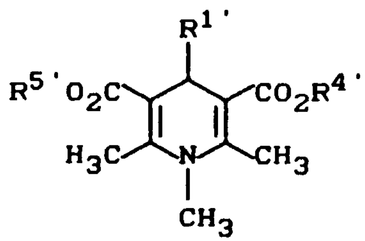

| Bsp.-Nr. | $R^{1'}$ | $R^{4'}$ | $R^{5'}$ | F° C | Ausbeute (% d.Th.) |
|---|---|---|---|---|---|
| 4 | 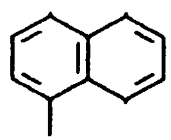 | $-C_2H_5$ | $-C_2H_5$ | 90-92 | 63,9 |

**Tabelle 2:**

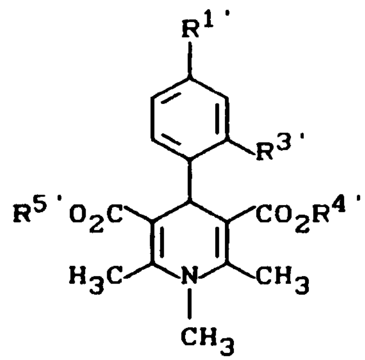

| Bsp.-Nr. | $R^{1'}$ | $R^{3'}$ | $R^{4'}$ | $R^{5'}$ | F°C | Ausbeute (% d.Th.) |
|---|---|---|---|---|---|---|
| 5 | H | CN | $-C_3H_7$ | $-C_3H_7$ | Öl | 19,4 |
| 6 | $NO_2$ | H | $-C_4H_9$ | $-C_4H_9$ | 81-84 | 62,7 |
| 7 | $CF_3$ | H | $-C_3H_7$ | $-CH_3$ | 76-78 | |
| 8 | $CF_3$ | H | $-CH(CH_3)_2$ | $-CH_3$ | 86-88 | |
| 9 | $CF_3$ | H | $-CH-CH(CH_3)_2$<br>\|<br>$CH_3$ | $-CH_3$ | 54-57 | |
| 10 | $CF_3$ | H | $-(CH_2)_2-OCH_3$ | $-CH_3$ | 71-73 | |

Beispiel 11:

4-(4-Bromphenyl)-1,2,6-trimethyl-1,4-dihydro-pyridin-3,5-dicarbonsäuredipropylester

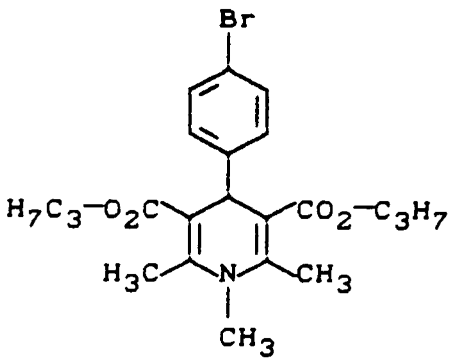

Eine Lösung von 6,22 g (0,02 mol) 2-(4-Brombenzyliden)-acetessigsäurepropylester und 3,14 g (0,02 mol) 3-Methylamino-crotonsäurepropylester in 25 ml 2-Butanol wird 10 Stunden unter Rückfluß gerührt. Dann wird im Vakuum eingeengt, der in der Kälte entstehende Niederschlag abgesaugt. Nach der Umkristallisation aus Propanol erhält man 5,32 g (59,1% der Theorie) vom Schmp. 97-99 °C.

Analog des Beispiels 11 lassen sich die in der Tabelle 3 aufgeführten Verbindungen herstellen.

**Tabelle 3:**

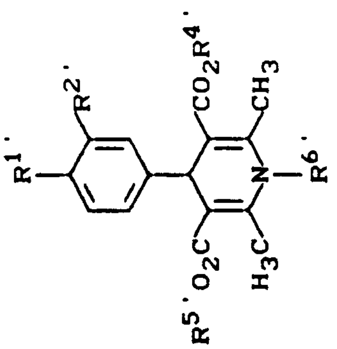

| Bsp.-Nr. | R1' | R2' | R4' | R5' | R6' | F° C | Ausbeute (% d.Th.) |
|---|---|---|---|---|---|---|---|
| 12 | H | $-CH_3$ | $-C_3H_7$ | $-C_3H_7$ | $-CH_3$ | 67-68 | 23,3 |
| 13 | -Br | H | $-CH_3$ | $-CH_3$ | $-CH_3$ | 163-167 | 59,5 |
| 14 | -Br | H | $-C_4H_9$ | $-C_4H_9$ | $-CH_3$ | 66-68 | 36,5 |
| 15 | -CN | H | $-C_3H_7$ | $-C_3H_7$ | $-CH_3$ | 116-119 | 45,7 |
| 16 | H | $-CF_3$ | $-C_2H_5$ | $-C_2H_5$ | 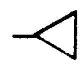 | 112-114 | 22,4 |
| 17 | $-CF_3$ | H | $-CH_3$ | $-CH_3$ | $-C_2H_5$ | 119-129 | 15,5 |
| 18 | $-CF_3$ | H | $-CH_3$ | $-CH_3$ | 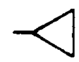 | 137-138 | 15,6 |

Fortsetzung Tabelle 3

| Bsp.-Nr. | $R^{1'}$ | $R^{2'}$ | $R^{4'}$ | $R^{5'}$ | $R^{6'}$ | F° C | Ausbeute (% d.Th.) |
|---|---|---|---|---|---|---|---|
| 19 | $-CF_3$ | H | $-C_2H_5$ | $-C_2H_5$ | 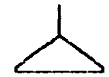 | 111-113 | 15,5 |
| 20 | $-CF_3$ | H | $-CH(CH_3)_2$ | $-CH(CH_3)_2$ | $-CH_3$ | 106-108 | 49,1 |
| 21 | $-CH_3$ | $-NO_2$ | $-CH_3$ | $-CH_3$ | $-CH_3$ | 116-118 | 66,1 |
| 22 | $-CH_3$ | $-NO_2$ | $-C_2H_5$ | $-C_2H_5$ | $-CH_3$ | 97-98 | 60,1 |
| 23 | $-CH_3$ | $-NO_2$ | $-C_3H_7$ | $-C_3H_7$ | $-CH_3$ | 94-96 | 41,3 |
| 24 | $-CH_3$ | $-NO_2$ | $-C_4H_9$ | $-C_4H_9$ | $-CH_3$ | 89-90 | 45,4 |
| 25 | $-NO_2$ | Cl | $-C_2H_5$ | $-C_2H_5$ | $-CH_3$ | 128-130 | 60,1 |
| 26 | -Cl | $-CF_3$ | $-CH_3$ | $-CH_3$ | $-CH_3$ | 122-124 | 63,8 |
| 27 | $-CH_3$ | $-CF_3$ | $-CH_3$ | $-CH_3$ | $-CH_3$ | 103-105 | 4,83 |
| 28 | $-NO_2$ | -OH | $-CH_3$ | $-CH_3$ | $-CH_3$ | 187-189 | 62,6 |
| 29 | $-OCF_3$ | H | $-CH_3$ | $-CH_3$ | 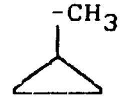 | 77-78 | 13,2 |
| 30 | $-OCF_3$ | H | $-C_2H_5$ | $-C_2H_5$ | 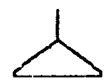 | 108-109 | 18,5 |
| 31 | $-CF_3$ | H | $-CH_2-CH_2-OCH_3$ | $-CH(CH_3)_2$ | $-CH_3$ | Öl | 74,3 |
| 31a | (+)-Enantiomer $[\alpha]^{20}_{589}$ 10,3 (C = 1 in $CHCl_3$) | | | | | | |
| 31b | (-)-Enantiomer $[\alpha]^{20}_{589}$ -10,1 (C = 1 in $CHCl_3$) | | | | | | |

Beispiel 32

1,2,6-Trimethyl-4-(4-trifluormethylphenyl)-1,4-dihydropyridin-3,5-dicarbonsäurediethylester

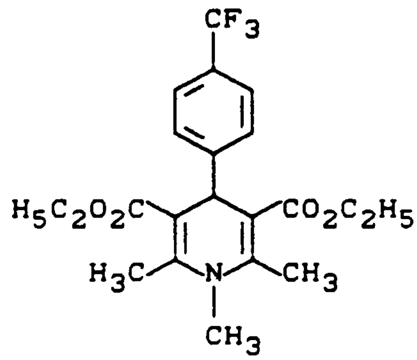

Unter Stickstoffschutz fügt man zu 20 ml Toluol 0,55 ml (5 mmol) Titantetrachlorid, anschließend 1 ml (10 mmol) Piperidin und rührt 5 min. Nach dem Zutropfen von 2,9 g (20 mmol) 3-Methylaminocrotonsäuremethylester fügt man 1,36 ml (10 mmol) 4-Trifluormethylphenylbenzaldehyd zu und rührt 3 Stunden bei Raumtemperatur. Zur Aufarbeitung wird 100 ml 5%ige Salzsäure zugesetzt und die organische Phase mit Essigester aufgenommen, die Essigesterlösung nacheinander mit 5% Salzsäure und mit Natriumbicarbonatlösung gewaschen. Nach dem Trocknen der Essigesterlösung über Natriumsulfat, Eindampfen und Verrühren des Rückstandes in n-Heptan erhält man 1,7 g (41,4% der Theorie)
Schmp.: 98 °C

Beispiel 33

1-Cyclopropyl-2,6-dimethyl-4-(4-trifluormethylphenyl)-1,4-dihydro-pyridin-3,5-dicarbonsäure-methylethylester

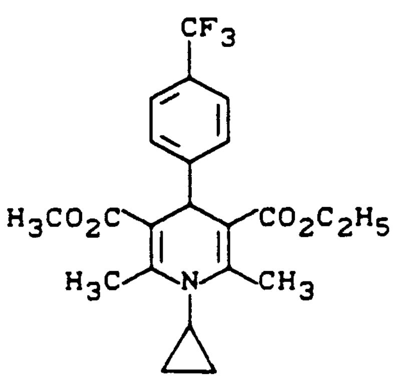

Zu einer Lösung von 3.9 g (0,03 mol) Acetessigsäureethylester und 8,16 g (0,03 mol) 2-(4-Trifluormethylbenzyliden)-acetessigsäuremethylesterin 50 ml Pyridin fügt man 3,6 g (0,039 mol) Cyclopropylaminhydrochlorid und erhitzt 5 Stunden unter Rückfluß. Man engt das Reaktionprodukt im Vakuum ein, nimmt den Rückstand in Methylenchlorid und Wasser auf, trennt die wäßrige Phase ab, trocknet die Methylenchloridlösung über Natriumsulfat und dampft ein. Der Rückstand wird durch Chromatographie über Kieselgel mit Methylenchlorid als Lösemittel gereinigt. Nach dem Umlösen aus n-Heptan erhält man 2,2 g (17,3% der Theorie).
Schmp.: 110 °C
In Analogie zur Vorschrift der Beispiele 32 und 33 können die in Tabelle 4 aufgeführten Verbindungen hergestellt werden.

**Tabelle 4:**

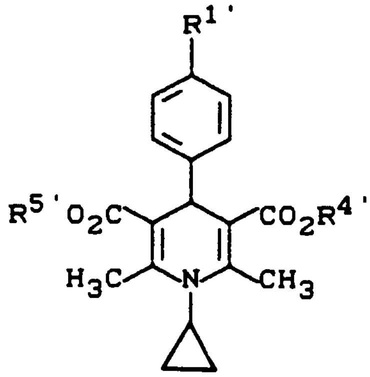

| Bsp.-Nr. | $R^{1'}$ | $R^{4'}$ | $R^{5'}$ | F° C |
|---|---|---|---|---|
| 34 | $-CF_3$ | $-C_4H_9$ | $-C_3H_7$ | 62 |
| 35 | $-CF_3$ | $-(CH_2)_2OCH_3$ | $-C_3H_7$ | Öl |
| 36 | -F | $-CH_3$ | $-CH_3$ | 140 |
| 37 | -F | $-C_2H_5$ | $-C_2H_5$ | 79 |
| 38 | $-CF_3$ | $-C_4H_9$ | $-CH_3$ | Öl |
| 39 | $-CF_3$ | $-C_3H_7$ | $-C_2H_5$ | 55-57 |
| 40 | $-CF_3$ | $-C_4H_9$ | $-C_2H_5$ | 55-60 |
| 41 | $-CF_3$ | $-C_2H_5$ | $-CH(CH_3)_2$ | 96 |

Die in der Tabelle 5 aufgeführten Beispiele wurden in Analogie zu den Vorschriften dar Beispiele 1, 3 und 11 hergestellt.

Tabelle 5:

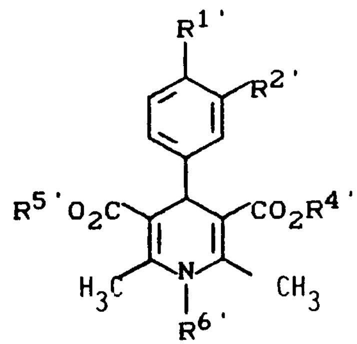

| Beispiel Nr. | $R^{1'}$ | $R^{2'}$ | $R^{4'}$ | $R^{5'}$ | $R^{6'}$ | F° C | Ausbeute (% d. Th.) |
|---|---|---|---|---|---|---|---|
| 42 | $-CF_3$ | H | $-CH_3$ | $-C_2H_5$ | $-CH_3$ | 116-118 | 78 |
| 43 | -F | H | $-CH_3$ | $-CH(CH_3)_2$ | $-CH_3$ | 92-94 | 73 |
| 44 | -F | H | $C_2H_5$ | $-CH(CH_3)_2$ | $-CH_3$ | Öl | 73 |
| 45 | $-CF_3$ | H | $-(CH_2)_2OCH_3$ | $-C_2H_5$ | $-CH_3$ | 56-57 | 57 |
| 46 | $-CF_3$ | H | $-(CH_2)_2OCH_3$ | $CH_2-CH(CH_3)_2$ | $-CH_3$ | 55-60 | 68 |
| 47 | -F | $-CF_3$ | $-CH_3$ | $-CH_3$ | $-CH_3$ | 133 | 59 |
| 48 | -F | $-CF_3$ | $-C_2H_5$ | $-C_2H_5$ | 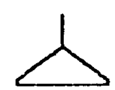 | 61 | 19 |

Tabelle 5: Fortsetzung

| Beispiel Nr. | $R^{1'}$ | $R^{2'}$ | $R^{4'}$ | $R^{5'}$ | $R^{6'}$ | F° C | Ausbeute (% d. Th.) |
|---|---|---|---|---|---|---|---|
| 49 | $-CF_3$ | H | $-CH_3$ | $-CH_2-CH(CH_3)_2$ | $-CH_3$ | 68 | 58 |
| 50 | $-CF_3$ | H | $-C_2H_5$ | $-CH_2-CH(CH_3)_2$ | $-CH_3$ | 63 | 75 |
| 51 | -F | $-CF_3$ | $-CH_3$ | $-CH_3$ | 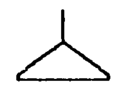 | 118 | 27 |
| 52 | -F | $-CF_3$ | $-C_2H_5$ | $-C_2H_5$ | $-CH_3$ | 83 | 63 |
| 53 | $-OCF_3$ | H | $-(CH_2)_2OCH_3$ | $-CH_2CH(CH_3)_2$ | $-CH_3$ | 62 | 21 |
| 54 | $-OCF_3$ | H | $-C_2H_5$ | $-CH_2CH(CH_3)_2$ | $-CH_3$ | 73 | 19 |
| 55 | $-CF_3$ | H | $-(CH_2)_2OCH_3$ | $-CH_2-CH(CH_3)_2$ | $-CH_3$ | Öl (-) Enant. α20 589 :-11,1 ($CHCl_3$), c:0,85 | 45 |
| 56 | -CN | H | $-CH_3$ | $-CH(CH_3)_2$ | $-CH_3$ | 122 | 50 |
| 57 | $-CF_3$ | H | $-CH_2CH(CH_3)_2$ | $-CH_2CH(CH_3)_2$ | $-CH_3$ | 68 | 45 |

**Tabelle 5**: Fortsetzung

| Beispiel Nr. | $R^{1'}$ | $R^{2'}$ | $R^{4'}$ | $R^{5'}$ | $R^{6'}$ | F° C | Ausbeute (% d. Th.) |
|---|---|---|---|---|---|---|---|
| 58 | -CN | H | $-C_2H_5$ | $-CH(CH_3)_2$ | $-CH_3$ | 106-107 | 50 |
| 59 | $-CF_3$ | H | $-CH_2CH(CH_3)_2$ | $-CH_2CH(CH_3)_2$ | 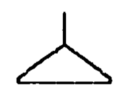 | 92 | 11 |
| 60 | -F | $-CF_3$ | $-(CH_2)_2OCH_3$ | $-CH(CH_3)_2$ | $-CH_3$ | 82-83 | 25 |
| 61 | $-CF_3$ | H | $-C_6H_{13}$ | $-CH_3$ | $-CH_3$ | 64-65 | 36 |
| 62 | $-CF_3$ | H | $-C_4H_9$ | $-CH_3$ | $-CH_3$ | 71-73 | 28 |
| 63 | $-CF_3$ | H | $-C_5H_{11}$ | $-CH_3$ | $-CH_3$ | 75-77 | 13 |
| 64 | -Cl | H | $-CH_3$ | $-CH_3$ | $-C_2H_5$ | 143 | 21 |
| 65 | -Cl | H | $-CH_3$ | $-CH_3$ | $-CH_3$ | 181 | 19 |

**Patentansprüche**

**Patentansprüche für folgende Vertragsstaaten : AT, BE, CH, DE, DK, FR, GB, IT, LI, LU, NL, SE**

1. Verwendung von N-alkylierten 1,4-Dihydropyridinen der allgemeinen Formel (I)

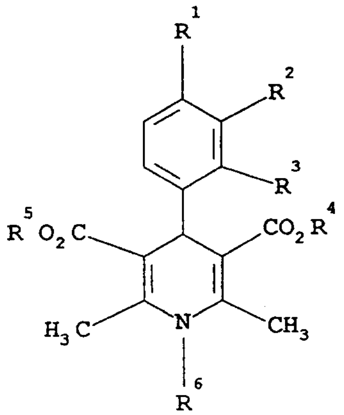

(I)

in welcher

| | |
|---|---|
| $R^1$ | für Wasserstoff, Nitro, Trifluormethyl, Trifluormethoxy, Cyano, Fluor, Chlor Brom oder Methyl steht, |
| $R^2$ | für Wasserstoff, Fluor, Chlor, Brom, Nitro, Hydroxy, Trifluormethyl oder Methyl steht, |
| $R^3$ | für Wasserstoff oder Cyano steht, oder |
| $R^2$ und $R^3$ | gemeinsam einen ankondensierten Benzoring bilden, |
| $R^4$ und $R^5$ | gleich oder verschieden sind und für geradkettiges oder verzweigtes Alkyl mit bis zu 6 Kohlenstoffatomen stehen, das gegebenenfalls durch Methoxy substituiert ist, und |
| $R^6$ | für geradkettiges oder verzweigtes Alkyl mit bis zu 8 Kohlenstoffatomen oder für Cyclopropyl steht, |

zur Herstellung eines hämorheologischen Arzneimittels zur Behandlung von akuten und chronischen ischämischen Erkrankungen durch Verbesserung der Blutviskosität durch Beeinflussung der Erythrozyten und Leukozyten bei gleichzeitigem Fehlen einer blutdrucksenkenden Wirkung.

2. Verwendung von N-alkylierten 1,4-Dihydropyridinen der allgemeinen Formel (I), in welcher

| | |
|---|---|
| $R^1$ | für Fluor, Brom oder Trifluormethyl steht, |
| $R^2$ und $R^3$ | jeweils für Wasserstoff stehen, |
| $R^4$ und $R^5$ | gleich oder verschieden sind und für geradkettiges oder verzweigtes Alkyl mit bis zu 6 Kohlenstoffatomen stehen, das gegebenenfalls durch Methoxy substituiert ist, und |
| $R^6$ | für geradkettiges oder verzweigtes Alkyl mit bis zu 8 Kohlenstoffatomen oder für Cyclopropyl steht, |

zur Herstellung eines hämorheologischen Arzneimittels gemäß Anspruch 1.

3. Verwendung von Verbindungen der allgemeinen Formel I gemäß Anspruch 1 zur Herstellung eines hämorheologischen Arzneimittels zur Behandlung von ischämischen Erkrankungen deren blutdrucksenkende Dosis mindestens um den Faktor 10 höher liegt als die hämorheologisch wirksame Dosis.

4. Verwendung von N-alkylierten 1,4-Dihydropyridinen der allgemeinen Formel (I) gemäß Anspruch 1 zur Herstellung eines hämorheologisch wirkenden Arzneimittels zur Behandlung von Claudicatio intermittens, Myocardinfarkt, Gehirninfarkt und Reperfusionsschäden.

5. N-alkylierte 1,4-Dihydropyridine aus der Gruppe
1,2,6-Trimethyl-4-(4-fluorphenyl)-1,4-dihydropyridin-3,5-dicarbonsäurediethylester
1,2,6-Trimethyl-4-(4-trifluormethylphenyl)-1,4-dihydropyridin-3,5-dicarbonsäuredimethylester
1,2,6-Trimethyl-4-(4-trifluormethylphenyl)-1,4-dihydropyridin-3,5-dicarbonsäure-methyl-propylester
1,2,6-Trimethyl-4-(4-trifluormethylphenyl)-1,4-dihydropyridin-3,5-dicarbonsäure-methyl-(1,2-dimethylpropyl)ester

1,2,6-Trimethyl-4-(4-trifluormethylphenyl)-1,4-dihydropyridin-3,5-dicarbonsäure-methyl-(2-methoxyethyl)-ester
1,2,6-Trimethyl-4-(4-bromphenyl)-1,4-dihydropyridin-3,5-dicarbonsäuredimethylester
1,2,6-Trimethyl-4-(4-bromphenyl)-1,4-dihydropyridin-3,5-dicarbonsäuredipropylester
1,2,6-Trimethyl-4-(4-bromphenyl)-1,4-dihydropyridin-3,5-dicarbonsäuredibutylester
1-Cyclopropyl-2,6-dimethyl-4-(3-trifluormethylphenyl)-1,4-dihydropyridin-3,5-dicarbonsäurediethylester
1-Ethyl-2,6-dimethyl-4-(4-trifluormethylphenyl)-1,4-dihydropyridin-3,5-dicarbonsäuredimethylester
1-Cyclopropyl-2,6-dimethyl-4-(4-trifluormethylphenyl)-1,4-dihydropyridin-3,5-dicarbonsäuredimethylester
1-Cyclopropy-2,6-dimethyl-4-(4-trifluormethylphenyl)-1,4-dihydropyridin-3,5-dicarbonsäurediethylester
1,2,6-Trimethyl-4-(4-trifluormethylphenyl)-1,4-dihydropyridin-3,5-dicarbonsäurediisopropylester
1-Cyclopropyl-2,6-dimethyl-4-(4-trifluormethoxyphenyl)-1,4-dihydropyridin-3,5-dicarbonsäuredimethylester
1-Cyclopropyl-2,6-dimethyl-4-(4-trifluormethoxyphenyl)-1,4-hydropyridin-3,5-dicarbonsäurediethylester
1,2,6-Trimethyl-4-(4-trifluormethylphenyl)-1,4-dihydropyridin-3,5-dicarbonsäure-isopropyl-(2-methoxyethyl)-ester
1,2,6-Trimethyl-4-(4-trifluormethylphenyl)-1,4-dihydropyridin-3,5-dicarbonsäurediethylester
1-Cyclopropyl-2,6-dimethyl-4-(4-trifluormethylphenyl)-1,4-dihydropyridin-3,5-dicarbonsäuremethylethylester
1-Cyclopropyl-2,6-dimethyl-4-(4-trifluormethylphenyl)-1,4-dihydropyridin-3,5-dicarbonsäurepropyl-(2-methoxyethyl)ester
1-Cyclopropyl-2,6-dimethyl-4-(4-trifluormethylphenyl)-3,5-dicarbonsäure-1,4-dihydropyridin-isopropyl-(2-methoxyethyl)ester
1-Cyclopropyl-2,6-dimethyl-4-(4-fluorphenyl)-1,4-dihydropyridin-3,5-dicarbonsäurediethylester
1-Cyclopropyl-2,6-dimethyl-4-(4-fluorphenyl)-1,4-dihydropyridin-3,5-dicarbonsäuredimethylester
1-Cyclopropyl-2,6-dimethyl-4-(4-trifluorphenyl)-1,4-dihydropyridin-3,5-dicarbonsäure-propyl-butylester
1-Cyclopropyl-2,6-dimethyl-4-(4-trifluormethylphenyl)-1,4-dihydropyridin-3,5-dicarbonsäurebutylmethylester
1-Cydopropyl-2,6-dimethyl-4-(4-trifluormethylphenyl)-1,4-dihydropyridin-3,5-dicarbonsäureethylpropylester
1-Cyclopropyl-2,6-dimethyl-4-(4-trifluormethylphenyl)-1,4-dihydropyridin-3,5-dicarbonsäurebutylethylester
1-Cyclopropyl-2,6-dimethyl-4-(4-trifluormethylphenyl)-1,4-dihydropyridin-3,5-dicarbonsäureethylisopropylester.

**6.** Verfahren zur Herstellung von neuen Dihydropyridinverbindungen gemäß Anspruch 5, dadurch gekennzeichnet, daß man

[A] Benzylidenverbindungen der allgemeinen Formel II

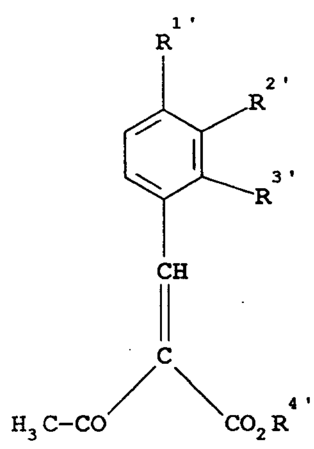

(II)

in welcher

$R^{1'}$, $R^{2'}$, $R^{3'}$ und $R^{4'}$ die oben angegebene Bedeutung von $R^1$, $R^2$, $R^3$ und $R^4$ haben und außerden den jeweiligen Bedeutungsumfang der in Anspruch 5 aufgeführten neuen Verbindungen erfassen, entweder zunächst mit β-Aminocrotonsäureestern der allgemeinen Formel (III)

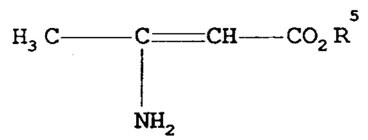
(III)

in welcher $R^{5'}$ den Bedeutungsumfang der in Anspruch 5 aufgeführten neuen Verbindungen erfaßt,
in inerten Lösemitteln umsetzt und in einem letzten Schritt nach üblicher Methode die -NH-Funktion alkyliert, oder direkt die Verbindungen der allgemeinen Formel (II) gegebenenfalls in Anwesenheit von Lewissäuren wie Titantetrachlorid mit Verbindungen der allgemeinen Formel (IIIa)

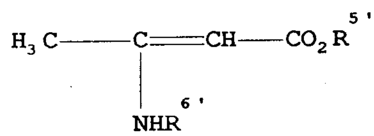
(IIIa)

in welcher
$R^{5'}$ die oben angegebene Bedeutung hat,
$R^{6'}$ die in Anspruch 5 angegebene Bedeutung von $R^6$ hat und außerdem den Bedeutungsumfang der dort aufgeführten neuen Verbindungen erfaßt,
umsetzt, oder
[B] Aldehyde der allgemeinen Formel (IV)

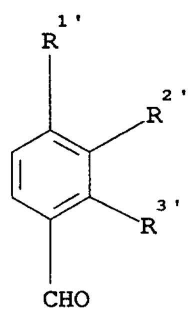
(IV)

in welcher
$R^{1'}$, $R^{2'}$, $R^{3'}$ die unter Verfahren [A] angegebene Bedeutung haben,
zunächst mit β-Ketocarobnsäureestern der allgemeinen Formeln (V) und (Va)

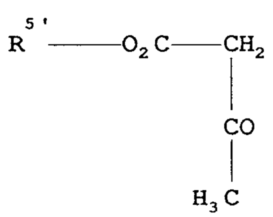
(V) und
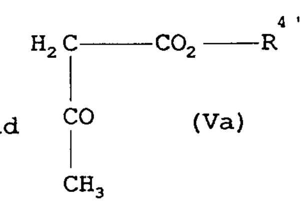
(Va)

in welcher
$R^{4'}$ und $R^{5'}$ ebenfalls die unter Verfahren [A] angegebene Bedeutung haben, umsetzt und anschließend entweder direkt mit Aminen oder den entsprechenden Aminhydrochloriden der allgemeinen Formel (VI)

$H_2N—R^{6'}$ (VI)

in welcher $R^{6'}$ die oben unter Verfahren [A] angegebene Bedeutung hat, umsetzt oder zunächst mit Ammoniak in organischen, gegebenenfalls inerten Lösemitteln nach üblicher Methode ringschließt und in einem letzten Schritt nach der oben aufgeführten Methode alkyliert,
und im Fall der enantiomerenreinen Ester zunächst die enantiomerenreinen Carbonsäuren herstellt und diese, gegebenenfalls über ein reaktives Säurederivat mit den entsprechenden Alkoholen nach üblicher Methode verestert.

**7.** Arzneimittel enthaltend mindestens eine Dihydropyridinverbindung gemäß Anspruch 5.

**8.** Verfahren zur Herstellung von Arzneimitteln, dadurch gekennzeichnet, daß man mindestens eine der in Anspruch 1 genannten Dihydropyridinverbindungen gegebenenfalls mit üblichen Hilfs- und Trägerstoffen in eine geeignete Applikationsform überführt.

**Patentansprüche für folgende Vertragsstaaten : ES, GR**

**1.** Verfahren zur Herstellung von neuen N-alkylierten 1,4-Dihydropyridinverbindungen der Formel (I)

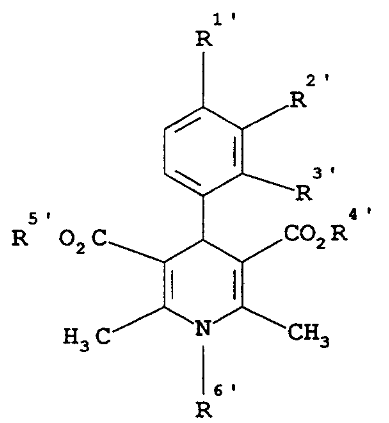

(I)

in welcher
$R^{1'}$ bis $R^{6'}$ die jeweilige Bedeutung gemäß den einzelnen Verbindungen der folgenden Gruppen haben,
1,2,6-Trimethyl-4-(4-fluorphenyl)-1,4-dihydropyridin-3,5-dicarbonsäurediethylester
1,2,6-Trimethyl-4-(4-trifluormethylphenyl)-1,4-dihydropyridin-3,5-dicarbonsäuredimethylester
1,2,6-Trimethyl-4-(4-trifluormethylphenyl)-1,4-dihydropyridin-3,5-dicarbonsäure-methyl-propylester
1,2,6-Trimethyl-4-(4-trifluormethylphenyl)-1,4-dihydropyridin-3,5-dicarbonsäure-methyl-(1,2-dimethylpropyl)ester
1,2,6-Trimethyl-4-(4-trifluormethylphenyl)-1,4-dihydropyridin-3,5-dicarbonsäure-methyl-(2-methoxyethyl)-ester
1,2,6-Trimethyl-4-(4-bromphenyl)-1,4-dihydropyridin-3,5-dicarbonsäuredimethylester
1,2,6-Trimethyl-4-(4-bromphenyl)-1,4-dihydropyridin-3,5-dicarbonsäuredipropylester
1,2,6-Trimethyl-4-(4-bromphenyl)-1,4-dihydropyridin-3,5-dicarbonsäuredibutylester
1-Cyclopropyl-2,6-dimethyl-4-(3-trifluormethylphenyl)-1,4-dihydropyridin-3,5-dicarbonsäurediethylester
1-Ethyl-2,6-dimethyl-4-(4-trifluormethylphenyl)-1,4-dihydropyridin-3,5-dicarbonsäuredimethylester
1-Cyclopropyl-2,6-dimethyl-4-(4-trifluormethylphenyl)-1,4-dihydropyridin-3,5-dicarbonsäuredimethylester
1-Cyclopropyl-2,6-dimethyl-4-(4-trifluormethylphenyl)-1,4-dihydropyridin-3,5-dicarbonsäurediethylester
1,2,6-Trimethyl-4-(4-trifluormethylphenyl)-1,4-dihydropyridin-3,5-dicarbonsäurediisopropylester
1-Cyclopropyl-2,6-dimethyl-4-(4-trifluormethoxyphenyl)-1,4-dihydropyridin-3,5-dicarbonsäuredimethylester
1-Cyclopropyl-2,6-dimethyl-4-(4-trifluormethoxyphenyl)-1,4-hydropyridin-3,5-dicarbonsäurediethylester
1,2,6-Trimethyl-4-(4-trifluormethylphenyl)-1,4-dihydropyridin-3,5-dicarbonsäure-isopropyl-(2-methoxyethyl)-ester
1,2,6-Trimethyl-4-(4-trifluormethylphenyl)-1,4-dihydropyridin-3,5-dicarbonsäurediethylester
1-Cyclopropyl-2,6-dimethyl-4-(4-trifluormethylphenyl)-1,4-dihydropyridin-3,5-dicarbonsäuremethylethylester
1-Cyclopropyl-2,6-dimethyl-4-(4-trifluormethylphenyl)-1,4-dihydropyridin-3,5-dicarbonsäurepropyl-(2-methoxyethyl)ester
1-Cyclopropyl-2,6-dimethyl-4-(4-trifluormethylphenyl)-3,5-dicarbonsäure-1,4-dihydropyridin-isopropyl-(2-methoxyethyl)ester
1-Cyclopropyl-2,6-dimethyl-4-(4-fluorphenyl)-1,4-dihydropyridin-3,5-dicarbonsäurediethylester
1-Cyclopropyl-2,6-dimethyl-4-(4-fluorphenyl)-1,4-dihydropyridin-3,5-dicarbonsäuredimethylester
1-Cyclopropyl-2,6-dimethyl-4-(4-trifluorphenyl)-1,4-dihydropyndin-3,5-dicarbonsäure-propyl-butylester
1-Cyclopropyl-2,6-dimethyl-4-(4-trifluormethylphenyl)-1,4-dihydropyridin-3,5-dicarbonsäurebutylmethylester
1-Cyclopropyl-2,6-dimethyl-4-(4-trifluormethylphenyl)-1,4-dihydropyridin-3,5-dicarbonsäureethylpropylester
1-Cyclopropyl-2,6-dimethyl-4-(4-trifluormethylphenyl)-1,4-dihydropyridin-3,5-dicarbonsäurebutylethylester
1-Cyclopropyl-2,6-dimethyl-4-(4-trifluormethylphenyl)-1,4-dihydropyridin-3,5-dicarbonsäureethylisopropylester,
dadurch gekennzeichnet, daß man

[A] Benzylidenverbindungen der allgemeinen Formel II

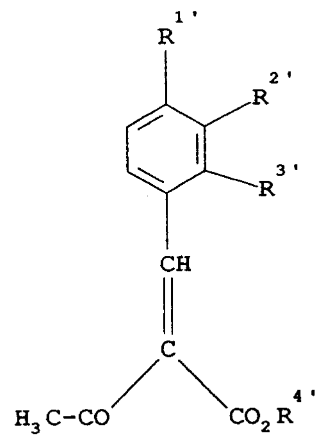

(II)

in welcher

$R^{1'}$, $R^{2'}$, $R^{3'}$ und $R^{4'}$ den jeweiligen Bedeutungsumfang der oben aufgeführten neuen Verbindungen haben,

entweder zunächst mit $\beta$-Aminocrotonsäureestern der allgemeinen Formel (III)

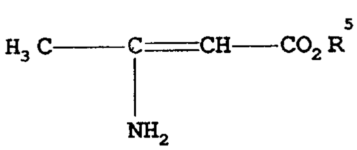

(III)

in welcher $R^{5'}$ den Bedeutungsumfang der jeweils angegebenen neuen Verbindungen hat,

in inerten Lösemitteln umsetzt und in einem letzten Schritt nach üblicher Methode die -NH-Funktion alkyliert, oder direkt die Verbindungen der allgemeinen Formel (II) gegebenenfalls in Anwesenheit von Lewissäuren wie Titantetrachlorid mit Verbindungen der allgemeinen Formel (IIIa)

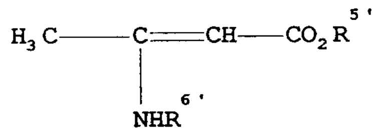

(IIIa)

in welcher

$R^{5'}$ und $R^{6'}$ den jeweiligen Bedeutungsumfang der oben angegebenen neuen Verbindungen haben,

umsetzt, oder

[B] Aldehyde der allgemeinen Formel (IV)

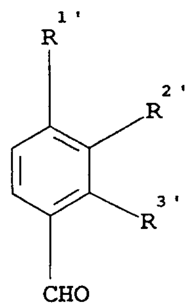

(IV)

in welcher

$R^{1'}$, $R^{2'}$, $R^{3'}$ die unter Verfahren [A] angegebene Bedeutung haben,

zunächst mit $\beta$-Ketocarobnsäureestern der allgemeinen Formeln (V) und (Va)

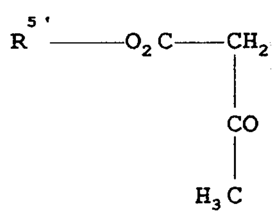

(V) und

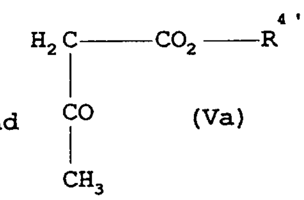

(Va)

in welcher

$R^{4'}$ und $R^{5'}$ ebenfalls die unter Verfahren [A] angegebene Bedeutung haben, umsetzt und anschließend entweder direkt mit Aminen oder den entsprechenden Aminhydrochloriden der allgemeinen Formel (VI)

$H_2N—R^{6'}$ (VI)

in welcher $R^{6'}$ die oben unter Verfahren [A] angegebene Bedeutung hat, umsetzt oder zunächst mit Ammoniak in organischen, gegebenenfalls inerten Lösemitteln nach üblicher Methode ringschließt und in einem letzten Schritt nach der oben aufgeführten Methode alkyliert,

und im Fall der enantiomerenreinen Ester zunächst die enantiomerenreinen Carbonsäuren herstellt und diese, gegebenenfalls über ein reaktives Säurederivat mit den entsprechenden Alkoholen nach üblicher Methode verestert.

**2.** Verfahren zur Herstellung von Arzneimitteln, dadurch gekennzeichnet, daß man mindestens eine der in Anspruch 1 genannten Dihydropyridinverbindungen gegebenenfalls mit üblichen Hilfs- und Trägerstoffen in eine geeignete Applikationsform überführt.

## Claims

**Claims for the following Contracting States : AT, BE, CH, DE, DK, FR, GB, IT, LI, LU, NL, SE**

**1.** Use of N-alkylated 1,4-dihydropyridines of the general formula (I)

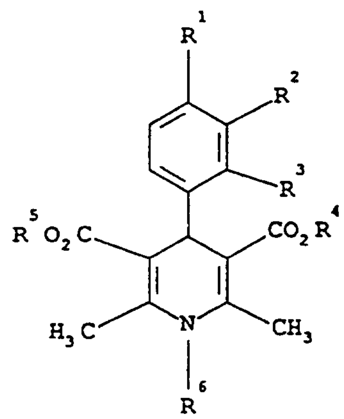

(I)

in which

| | |
|---|---|
| $R^1$ | represents hydrogen, nitro, trifluoromethyl, trifluoromethoxy, cyano, fluorine, chlorine, bromine or methyl, |
| $R^2$ | represents hydrogen, fluorine, chlorine, bromine, nitro, hydroxyl, trifluoromethyl or methyl, |
| $R^3$ | represents hydrogen or cyano, or |
| $R^2$ and $R^3$ | together form a fused benzo ring, |
| $R^4$ and $R^5$ | are identical or different and represent straight-chain or branched alkyl having up to 6 carbon atoms, which is optionally substituted by methoxy, and |
| $R^6$ | represents straight-chain or branched alkyl having up to 8 carbon atoms, or cyclopropyl, |

for the production of a haemorheological medicament for the treatment of acute and chronic ischaemic disorders by improving the blood viscosity by affecting the erythrocytes and leucocytes with simultaneous absence of a hypotensive action.

**2.** Use of N-alkylated 1,4-dihydropyridines of the general formula (I) in which

| | |
|---|---|
| $R^1$ | represents fluorine, bromine or trifluoromethyl, |
| $R^2$ and $R^3$ | in each case represent hydrogen, |
| $R^4$ and $R^5$ | are identical or different and represent straight-chain or branched alkyl having up to 6 carbon atoms, which is optionally substituted by methoxy, and |
| $R^6$ | represents straight-chain or branched alkyl having up to 8 carbon atoms, or cyclopropyl, |

for the production of a haemorheological medicament according to Claim 1.

**3.** Use of compounds of the general formula I according to Claim 1 for the production of a haemorheological medicament for the treatment of ischaemic disorders, the hypotensive dose of which is higher by at least the factor 10 than the haemorheologically active dose.

**4.** Use of N-alkylated 1,4-dihydropyridines of the general formula I according to Claim 1 for the production of a haemorheologically acting medicament for the treatment of intermittent claudication, myocardial infarct, cerebral infarct and reperfusion damage.

**5.** N-Alkylated 1,4-dihydropyridines from the group consisting of
diethyl 1,2,6-trimethyl-4-(4-fluorophenyl)-1,4-dihydropyridine-3,5-dicarboxylate
dimethyl 1,2,6-trimethyl-4-(4-trifluoromethylphenyl)-1,4-dihydropyridine-3,5-dicarboxylate
methyl propyl 1,2,6-trimethyl-4-(4-trifluoromethylphenyl)-1,4-dihydropyridine-3,5-dicarboxylate
methyl 1,2-dimethylpropyl 1,2,6-trimethyl-4-(4-trifluoromethylphenyl)-1,4-dihydropyridine-3,5-dicarboxylate

methyl 2-methoxyethyl 1,2,6-trimethyl-4-(4-trifluoromethylphenyl)-1,4-dihydropyridine-3,5-dicarboxylate
dimethyl 1,2,6-trimethyl-4-(4-bromophenyl)-1,4-dihydropyridine-3,5-dicarboxylate
dipropyl 1,2,6-trimethyl-4-(4-bromophenyl)-1,4-dihydropyridine-3,5-dicarboxylate
dibutyl 1,2,6-trimethyl-4-(4-bromophenyl)-1,4-dihydropyridine-3,5-dicarboxylate
diethyl 1-cyclopropyl-2,6-dimethyl-4-(3-trifluoromethylphenyl)-1,4-dihydropyridine-3,5-dicarboxylate
dimethyl 1-ethyl-2,6-dimethyl-4-(4-trifluoromethylphenyl)-1,4-dihydropyridine-3,5-dicarboxylate
dimethyl 1-cyclopropyl-2,6-dimethyl-4-(4-trifluoromethylphenyl)-1,4-dihydropyridine-3,5-dicarboxylate
diethyl 1-cyclopropyl-2,6-dimethyl-4-(4-trifluoromethylphenyl)-1,4-dihydropyridine-3,5-dicarboxylate
diisopropyl 1,2,6-trimethyl-4-(4-trifluoromethylphenyl)-1,4-dihydropyridine-3,5-dicarboxylate
dimethyl 1-cyclopropyl-2,6-dimethyl-4-(4-trifluoromethoxyphenyl)-1,4-dihydropyridine-3,5-dicarboxylate
diethyl 1-cyclopropyl-2,6-dimethyl-4-(4-trifluoromethoxyphenyl)-1,4-dihydropyridine-3,5-dicarboxylate
isopropyl 2-methoxyethyl 1,2,6-trimethyl-4-(4-trifluoromethylphenyl)-1,4-dihydropyridine-3,5-dicarboxylate
diethyl 1,2,6-trimethyl-4-(4-trifluoromethylphenyl)1,4-dihydropyridine-3,5-dicarboxylate
methyl ethyl 1-cyclopropyl-2,6-dimethyl-4-(4-trifluoromethylphenyl)-1,4-dihydropyridine-3,5-dicarboxylate
propyl 2-methoxyethyl 1-cyclopropyl-2,6-dimethyl-4-(4-trifluoromethylphenyl)-1,4-dihydropyridine-3,5-dicarboxylate
isopropyl 2-methoxyethyl 1-cyclopropyl-2,6-dimethyl4-(4-trifluoromethylphenyl)-1,4-dihydropyridine-3,5-dicarboxylate
diethyl 1-cyclopropyl-2,6-dimethyl-4-(4-fluorophenyl)-1,4-dihydropyridine-3,5-dicarboxylate
dimethyl 1-cyclopropyl-2,6-dimethyl-4-(4-fluorophenyl)-1,4-dihydropyridine-3,5-dicarboxylate
propyl butyl 1-cyclopropyl-2,6-dimethyl-4-(4-trifluorophenyl)-1,4-dihydropyridine-3,5-dicarboxylate
butyl methyl 1-cyclopropyl-2,6-dimethyl-4-(4-trifluoromethylphenyl)-1,4-dihydropyridine-3,5-dicarboxylate
ethyl propyl 1-cyclopropyl-2,6-dimethyl-4-(4-trifluoromethylphenyl)-1,4-dihydropyridine-3,5-dicarboxylate
butyl ethyl 1-cyclopropyl-2,6-dimethyl-4-(4-trifluoromethylphenyl)-1,4-dihydropyridine-3,5-dicarboxylate
ethyl isopropyl 1-cyclopropyl-2,6-dimethyl-4-(4-trifluoromethylphenyl)-1,4-dihydropyridine-3,5-dicarboxylate.

**6.** Process for the preparation of new dihydropyridine compounds according to Claim 5, characterized in that

[A] benzylidene compounds of the general formula II

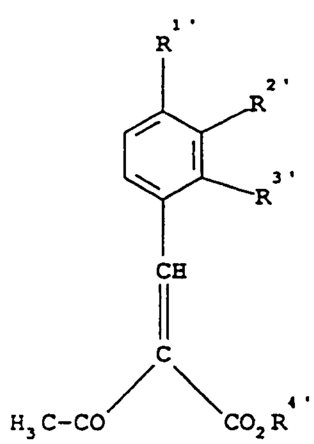

(II)

in which

$R^{1'}$, $R^{2'}$, $R^{3'}$ and $R^{4'}$ have the meaning of $R^1$, $R^2$, $R^3$ and $R^4$ indicated above and additionally cover the particular scope of meaning of the new compounds mentioned in Claim 5, are either reacted first with $\beta$-aminocrotonate esters of the general formula (III)

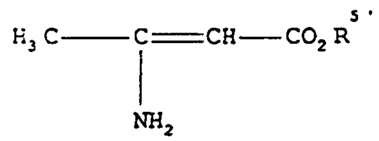 (III)

in which $R^{5'}$ covers the scope of meaning of the new compounds mentioned in Claim 5,
in inert solvents and in a last step the -NH function is alkylated according to a customary method, or the compounds of the general formula (II) are reacted directly, optionally in the presence of Lewis acids such as titanium tetrachloride, with compounds of the general formula (IIIa)

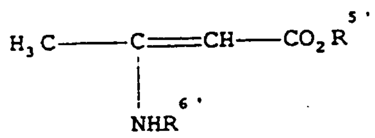 (IIIa)

in which

$R^{5'}$ has the meaning indicated above,

$R^{6'}$ has the meaning of $R^6$ indicated in Claim 5 and additionally covers the scope of meaning of the new compounds mentioned there, or

[B] aldehydes of the general formula (IV)

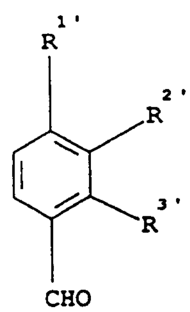 (IV)

in which

$R^{1'}$, $R^{2'}$, $R^{3'}$ have the meaning indicated under process [A],

are reacted first with $\beta$-ketocarboxylate esters of the general formulae (V) and (Va)

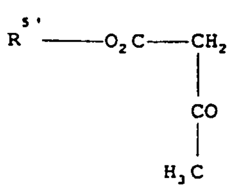 (V) and 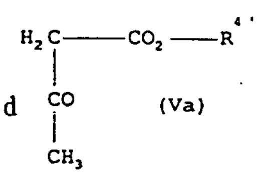 (Va)

in which

$R^{4'}$ and $R^{5'}$ likewise have the meaning indicated under process [A], and the products are then reacted either directly with amines or the corresponding amine hydrochlorides of the general formula (VI)

$H_2N—R^{6'}$ (VI)

in which $R^{6'}$ has the meaning indicated above under process [A], or first cyclized with ammonia in organic, optionally inert, solvents according to a customary method and alkylated in a last step

according to the abovementioned method,
and in the case of the enantiomerically pure esters the enantiomerically pure carboxylic acids are first prepared and these are esterified, optionally via a reactive acid derivative, with the appropriate alcohols according to a customary method.

**7.** Medicaments containing at least one dihydropyridine compound according to Claim 5.

**8.** Process for the production of medicaments, characterized in that at least one of the dihydropyridine compounds mentioned in Claim 1 is converted into a suitable administration form, if appropriate using customary auxiliaries and excipients.

**Claims for the following Contracting States : ES, GR**

**1.** Process for the preparation of new N-alkylated 1,4-dihydropyridine compounds of the formula (I)

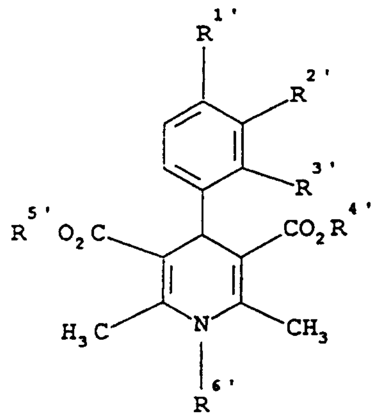

(I)

in which
$R^{1'}$ to $R^{6'}$ have the respective meaning as in the individual compounds of the following groups,
diethyl 1,2,6-trimethyl-4-(4-fluorophenyl)-1,4-dihydropyridine-3,5-dicarboxylate
dimethyl 1,2,6-trimethyl-4-(4-trifluoromethylphenyl)-1,4-dihydropyridine-3,5-dicarboxylate
methyl propyl 1,2,6-trimethyl-4-(4-trifluoromethylphenyl)-1,4-dihydropyridine-3,5-dicarboxylate
methyl 1,2-dimethylpropyl 1,2,6-trimethyl-4-(4-trifluoromethylphenyl)-1,4-dihydropyridine-3,5-dicarboxylate
methyl 2-methoxyethyl 1,2,6-trimethyl-4-(4-trifluoromethylphenyl)-1,4-dihydropyridine-3,5-dicarboxylate
dimethyl 1,2,6-trimethyl-4-(4-bromophenyl)-1,4-dihydropyridine-3,5-dicarboxylate
dipropyl 1,2,6-trimethyl-4-(4-bromophenyl)-1,4-dihydropyridine-3,5-dicarboxylate
dibutyl 1,2,6-trimethyl-4-(4-bromophenyl)-1,4-dihydropyridine-3,5-dicarboxylate
diethyl 1-cyclopropyl-2,6-dimethyl-4-(3-trifluoromethylphenyl)-1,4-dihydropyridine-3,5-dicarboxylate
dimethyl 1-ethyl-2,6-dimethyl-4-(4-trifluoromethylphenyl)-1,4-dihydropyridine-3,5-dicarboxylate
dimethyl 1-cyclopropyl-2,6-dimethyl-4-(4-trifluoromethylphenyl)-1,4-dihydropyridine-3,5-dicarboxylate
diethyl 1-cyclopropyl-2,6-dimethyl-4-(4-trifluoromethylphenyl)-1,4-dihydropyridine-3,5-dicarboxylate
diisopropyl 1,2,6-trimethyl-4-(4-trifluoromethylphenyl)-1,4-dihydropyridine-3,5-dicarboxylate
dimethyl 1-cyclopropyl-2,6-dimethyl-4-(4-trifluoromethoxyphenyl)-1,4-dihydropyridine-3,5-dicarboxylate
diethyl 1-cyclopropyl-2,6-dimethyl-4-(4-trifluoromethoxyphenyl)-1,4-dihydropyridine-3,5-dicarboxylate
isopropyl 2-methoxyethyl 1,2,6-trimethyl-4-(4-trifluoromethylphenyl)-1,4-dihydropyridine-3,5-dicarboxylate
diethyl 1,2,6-trimethyl-4-(4-trifluoromethylphenyl)-1,4-dihydropyridine-3,5-dicarboxylate
methyl ethyl 1-cyclopropyl-2,6-dimethyl-4-(4-trifluoromethylphenyl)-1,4-dihydropyridine-3,5-dicarboxylate
propyl 2-methoxyethyl 1-cyclopropyl-2,6-dimethyl-4-(4-trifluoromethylphenyl)-1,4-dihydropyridine-3,5-dicarboxylate
isopropyl 2-methoxyethyl 1-cyclopropyl-2,6-dimethyl -4-(4-trifluoromethylphenyl)-1,4-dihydropyridine-3,5-dicarboxylate
diethyl 1-cyclopropyl-2,6-dimethyl-4-(4-fluorophenyl)-1,4-dihydropyridine-3,5-dicarboxylate

dimethyl 1-cyclopropyl-2,6-dimethyl-4-(4-fluorophenyl)-1,4-dihydropyridine-3,5-dicarboxylate
propyl butyl 1-cyclopropyl-2,6-dimethyl-4-(4-trifluorophenyl)-1,4-dihydropyridine-3,5-dicarboxylate
butyl methyl 1-cyclopropyl-2,6-dimethyl-4-(4-trifluoromethylphenyl)-1,4-dihydropyridine-3,5-dicarboxylate
ethyl propyl 1-cyclopropyl-2,6-dimethyl-4-(4-trifluoromethylphenyl)-1,4-dihydropyridine-3,5-dicarboxylate
butyl ethyl 1-cyclopropyl-2,6-dimethyl-4-(4-trifluoromethylphenyl)-1,4-dihydropyridine-3,5-dicarboxylate
ethyl isopropyl 1-cyclopropyl-2,6-dimethyl-4-(4-trifluoromethylphenyl)-1,4-dihydropyridine-3,5-dicarboxylate.
characterized in that
[A] benzylidene compounds of the general formula II

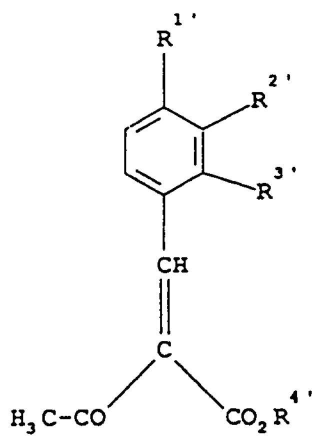

(II)

in which

$R^{1'}$, $R^{2'}$, $R^{3'}$ and $R^{4'}$ have the respective scope of meaning of the abovementioned new compounds,
are either reacted first with $\beta$-aminocrotonate esters of the general formula (III)

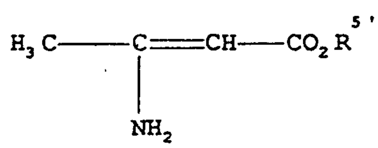

(III)

in which $R^{5'}$ has the scope of meaning of the new compounds indicated in each case,
in inert solvents and in a last step the -NH function is alkylated according to a customary method, or the compounds of the general formula (II) are reacted directly, optionally in the presence of Lewis acids such as titanium tetrachloride, with compounds of the general formula (IIIa)

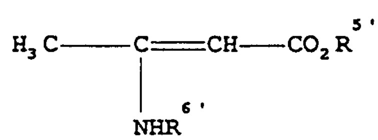

(IIIa)

in which

$R^{5'}$ and $R^{6'}$ have the respective scope of meaning of the new compounds indicated above, or

[B] aldehydes of the general formula (IV)

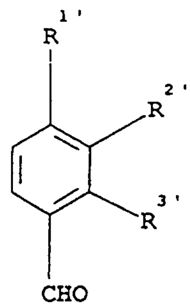

(IV)

in which

$R^{1'}$, $R^{2'}$, $R^{3'}$ have the meaning indicated under process [A],

are reacted first with $\beta$-ketocarboxylate esters of the general formulae (V) and (Va)

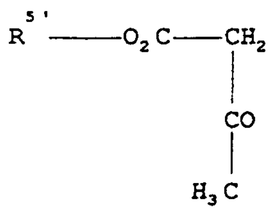

(V) and

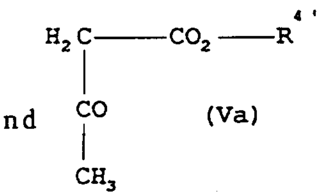

(Va)

in which

$R^{4'}$ and $R^{5'}$ likewise have the meaning indicated under process [A], and the products are then reacted directly with amines or the corresponding amine hydrochlorides of the general formula (VI)

$H_2N—R^{6'}$ (VI)

in which $R^{6'}$ has the meaning indicated above under process [A], or first cyclized with ammonia in organic, optionally inert solvents according to a customary method and alkylated in a last step according to the abovementioned method,

and in the case of the enantiomerically pure esters the enantiomerically pure carboxylic acids are first prepared and these are esterified, optionally via a reactive acid derivative, with the appropriate alcohols according to a customary method.

**2.** Process for the production of medicaments, characterized in that at least one of the dihydropyridine compounds mentioned in Claim 1 is converted into a suitable administration form, if appropriate using customary auxiliaries and excipients.

**Revendications**

**Revendications pour les Etats contractants suivant : AT, BE, CH, DE, DK, FR, GB, IT, LI, LU, NL, SE**

**1.** Utilisation de 1,4-dihydropyridines N-alkylées de formule générale (I)

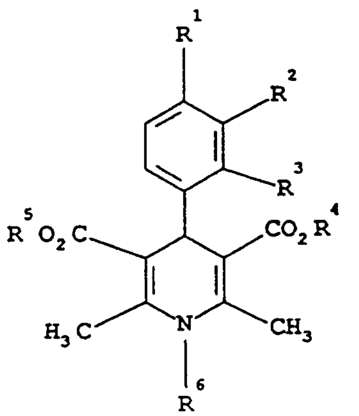

(I)

dans laquelle

| | |
|---|---|
| $R^1$ | représente de l'hydrogène, un groupe nitro, trifluorométhyle, trifluorométhoxy, cyano, du fluor, du chlore, du brome ou un groupe méthyle, |
| $R^2$ | est de l'hydrogène, du fluor, du chlore, du brome, un groupe nitro, hydroxy, trifluorométhyle ou méthyle, |
| $R^3$ | est de l'hydrogène ou un groupe cyano, ou bien |
| $R^2$ et $R^3$ | forment conjointement un noyau benzénique condensé, |
| $R^4$ et $R^5$ | sont identiques ou différents et représentent un groupe alkyle à chaîne droite ou ramifié ayant jusqu'à 6 atomes de carbone, qui est éventuellement substitué par un radical méthoxy, et |
| $R^6$ | est un groupe alkyle à chaîne droite ou ramifiée ayant jusqu'à 8 atomes de carbone ou un groupe cyclopropyle, |

pour la préparation d'un médicament hémorhéologique destiné au traitement de troubles ischémiques aigus et chroniques par amélioration de la viscosité sanguine sous l'influence exercée sur les érythrocytes et les leucocytes sans effet simultané d'abaissement de la pression sanguine.

**2.** Utilisation de 1,4-dihydropyridines N-alkylées de formule générale (I), dans laquelle

| | |
|---|---|
| $R^1$ | représente du fluor, du brome ou un groupe trifluorométhyle, |
| $R^2$ et $R^3$ | sont chacun de l'hydrogène, |
| $R^4$ et $R^5$ | sont identiques ou différents et représentent un groupe alkyle à chaîne droite ou ramifié ayant jusqu'à 6 atomes de carbone, qui est substitué le cas échéant par un radical méthoxy, et |
| $R^6$ | est un groupe alkyle à chaîne droite ou ramifié ayant jusqu'à 8 atomes de carbone ou un groupe cyclopropyle, |

pour la préparation d'un médicament hémorhéologique suivant la revendication 1.

**3.** Utilisation de composés de formule générale I suivant la revendication 1 pour la préparation d'un médicament hémorhéologique destiné au traitement de troubles ischémiques, dont la dose abaissant la pression sanguine est supérieure au moins d'un facteur 10 à la dose à effet hémorhéologique.

**4.** Utilisation de 1,4-dihydropyridines N-alkylées de formule générale (I) suivant la revendication 1 pour la préparation d'un médicament à action hémorhéologique destiné au traitement de la claudication intermittente, de l'infarctus du myocarde, de l'infarctus cérébral et des accidents de reperfusion.

**5.** 1,4-dihydropyridines N-alkylées du groupe des composés suivants :

ester diéthylique d'acide 1,2,6-triméthyl-4-(4-fluorophényl)-1,4-dihydropyridine-3,5-dicarboxylique

ester diméthylique d'acide 1,2,6-triméthyl-4-(4-trifluorométhylphényl)-1,4-dihydropyridine-3,5-dicarboxylique

ester de méthyle et de propyle d'acide 1,2,6-triméthyl-4-(4-trifluorométhylphényl)-1,4-dihydropyridine-3,5-dicarboxylique

ester de méthyle et de 1,2-diméthylpropyle d'acide 1,2,6-triméthyl-4-(4-trifluorométhylphényl)-1,4-dihydropyridine-3,5-dicarboxylique

ester de méthyle et de 2-méthoxyéthyle d'acide 1,2,6-triméthyl-4-(4-triflourométhylphényl)-1,4-dihydropyridine-3,5-dicarboxylique

ester diméthylique d'acide 1,2,6-triméthyl-4-(4-bromophényl)-1,4-dihydropyridine-3,5-dicarboxylique

ester dipropylique d'acide 1,2,6-triméthyl-4-(4-bromophényl)-1,4-dihydropyridine-3,5-dicarboxylique

ester dibutylique d'acide 1,2,6-triméthyl-4-(4-bromophényl)-1,4-dihydropyridine-3,5-dicarboxylique

ester diéthylique d'acide 1-cyclopropyl-2,6-diméthyl-4-(3-trifluorométhylphényl)-1,4-dihydropyridine-3,5-dicarboxylique

ester diméthylique d'acide 1-éthyl-2,6-diméthyl-4-(4-trifluorométhylphényl)-1,4-dihydropyridine-3,5-dicarboxylique

ester diméthylique d'acide 1-cyclopropyl-2,6-diméthyl-4-(4-trifluorométhylphényl)-1,4-dihydropyridine-3,5-dicarboxylique

ester diéthylique d'acide 1-cyclopropyl-2,6-diméthyl-4-(4-trifluorométhylphényl)-1,4-dihydropyridine-3,5-dicarboxylique

ester diisopropylique d'acide 1,2,6-triméthyl-4-(4-trifluorométhylphényl)-1,4-dihydropyridine-3,5-dicarboxylique

ester diméthylique d'acide 1-cyclopropyl-2,6-diméthyl-4-(4-trifluorométhoxyphényl)-1,4-dihydropyridine-3,5-dicarboxylique

ester diéthylique d'acide 1-cyclopropyl-2,6-diméthyl-4-(4-trifluorométhoxyphényl)-1,4-dihydropyridine-3,5-dicarboxylique

ester d'isopropyle et de 2-méthoxyéthyle d'acide 1,2,6-triméthyl-4-(4-trifluorométhylphényl)-1,4-dihydropyridine-3,5-dicarboxylique

ester diéthylique d'acide 1,2,6-triméthyl-4-(4-trifluorométhylphényl)-1,4-dihydropyridine-3,5-dicarboxylique

ester de méthyle et d'éthyle d'acide 1-cyclopropyl-2,6-diméthyl-4-(4-trifluorométhylphényl)-1,4-dihydropyridine-3,5-dicarboxylique

ester de propyle et de 2-méthoxyéthyle d'acide 1-cyclopropyl-2, 6-diméthyl-4-(4-trifluorométhylphényl)-1,4-dihydropyridine-3,5-dicarboxylique

ester d'isopropyle et de 2-méthoxyéthyle d'acide 1-cyclopropyl-2,6-diméthyl-4-(4-trifluorométhylphényl)-1,4-dihydropyridine-3,5-dicarboxylique

ester diéthylique d'acide 1-cyclopropyl-2,6-diméthyl-4-(4-fluorophényl)-1,4-dihydropyridine-3,5-dicarboxylique

ester diméthylique d'acide 1-cyclopropyl-2,6-diméthyl-4-(4-fluorophényl)-1,4-dihydropyridine-3,5-dicarboxylique

ester de propyle et de butyle d'acide 1-cyclopropyl-2,6-diméthyl-4-(4-trifluorophényl)-1,4-dihydropyridine-3,5-dicarboxylique

ester de butyle et de méthyle d'acide 1-cyclopropyl-2,6-diméthyl-4-(4-trifluorométhylphényl)-1,4-dihydropyridine-3,5-dicarboxylique

ester d'éthyle et de propyle d'acide 1-cyclopropyl-2,6-diméthyl-4-(4-trifluorométhylphényl)-1,4-dihydropyridine-3,5-dicarboxylique

ester de butyle et d'éthyle d'acide 1-cyclopropyl-2,6-diméthyl-4-(4-trifluorométhylphényl)-1,4-dihydropyridine-3,5-dicarboxylique

ester d'éthyle et d'isopropyle d'acide 1-cyclopropyl-2,6-diméthyl-4-(4-trifluorométhylphényl)-1,4-dihydropyridine-3,5-dicarboxylique.

**6.** Procédé de production de composés nouveaux de dihydropyridine suivant la revendication 5, caractérisé en ce que :

[A] on fait réagir des composés benzylidéniques de formule générale II

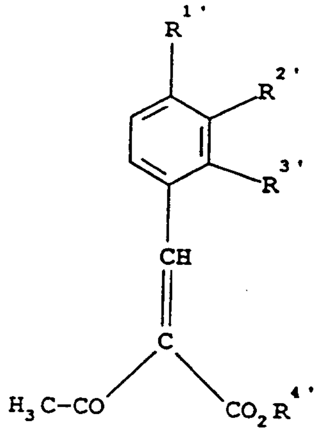

(II)

dans laquelle

$R^{1'}$, $R^{2'}$, $R^{3'}$ et $R^{4'}$ ont la définition indiquée ci-dessus pour $R^1$, $R^2$, $R^3$ et $R^4$ et englobent en outre les limites de définition de chacun des composés nouveaux indiqués dans la revendication 5, tout d'abord avec des esters d'acide $\beta$-aminocrotonique de formule générale (III)

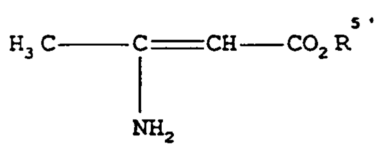

(III)

dans laquelle $R^{5'}$ comprend les limites de définition des composés nouveaux indiqués dans la revendication 5,

dans des solvants inertes et on alkyle dans une dernière étape la fonction -NH par un procédé classique, ou bien on fait réagir directement les composés de formule générale (II), le cas échéant en présence d'acides de Lewis tels que le tétrachlorure de titane, avec des composés de formule générale (IIIa)

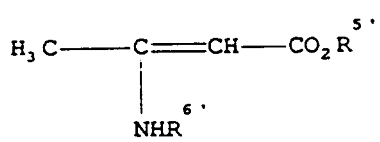

(IIIa)

dans laquelle

$R^{5'}$ a la définition indiquée ci-dessus,

$R^{6'}$ a la définition indiquée dans la revendication 5 pour $R^6$ et comprend en outre les limites de définition des composés nouveaux indiqués dans cette revendication, ou bien

[B] on fait réagir des aldéhydes de formule générale (IV)

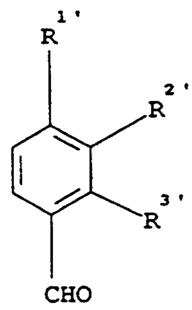

(IV)

dans laquelle
$R^{1'}$, $R^{2'}$, $R^{3'}$ ont la définition indiquée pour le procédé [A], tout d'abord avec des esters d'acides $\beta$-cétocarboxyliques de formules générales (V) et (Va)

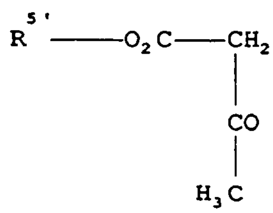

(V) et

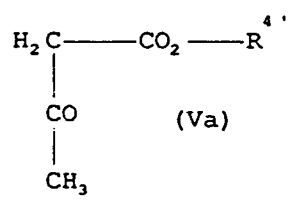

(Va)

dans lesquelles
$R^{4'}$ et $R^{5'}$ ont également la définition indiquée pour le procédé [A], puis on fait réagir directement le produit avec des amines ou les chlorhydrates d'amines correspondants de formule générale (VI)

$H_2N—R^{6'}$ (VI)

dans laquelle $R^{6'}$ a la définition indiquée ci-dessus pour le procédé [A], ou bien on le cyclise tout d'abord avec de l'ammoniac dans des solvants organiques éventuellement inertes, par un procédé classique, et on l'alkyle dans la dernière étape par le procédé indiqué ci-dessus, et dans le cas des esters de pureté énantiomérique, on prépare tout d'abord les acides carboxyliques de pureté énantiomérique et on les estérifie par un procédé classique avec les alcools correspondants, en passant éventuellement par un dérivé d'acide réactif.

**7.** Médicament contenant au moins un composé de dihydropyridine suivant la revendication 5.

**8.** Procédé de préparation de médicaments, caractérisé en ce qu'on fait prendre une forme administrable appropriée à au moins l'un des composés de dihydropyridine mentionnés dans la revendication 1, éventuellement avec des substances auxiliaires et des supports classiques.

**Revendications pour les Etats contractants suivants : ES, GR**

1. Procédé de production de composés nouveaux de 1,4-dihydropyridine N-alkylés de formule (I)

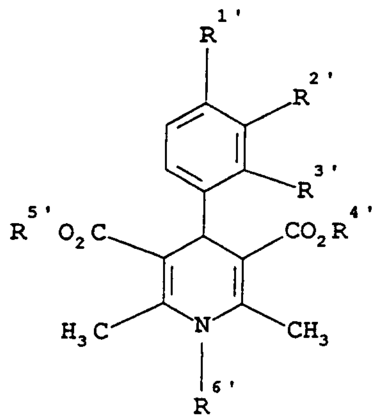

(I)

dans laquelle

$R^{1'}$ à $R^{6'}$ ont chacun la définition conforme aux composés individuels des groupes suivants,

ester diéthylique d'acide 1,2,6-triméthyl-4-(4-fluorophényl)-1,4-dihydropyridine-3,5-dicarboxylique

ester diméthylique d'acide 1,2,6-triméthyl-4-(4-trifluorométhylphényl)-1,4-dihydropyridine-3,5-dicarboxylique

ester de méthyle et de propyle d'acide 1,2,6-triméthyl-4-(4-trifluorométhylphényl)-1,4-dihydropyridine-3,5-dicarboxylique

ester de méthyle et de 1,2-diméthylpropyle d'acide 1,2,6-triméthyl-4-(4-trifluorométhylphényl)-1,4-dihydropyridine-3,5-dicarboxylique

ester de méthyle et de 2-méthoxyéthyle d'acide 1,2,6-triméthyl-4-(4-trifluorométhylphényl)-1,4-dihydropyridine-3,5-dicarboxylique

ester diméthylique d'acide 1,2,6-triméthyl-4-(4-bromophényl)-1,4-dihydropyridine-3,5-dicarboxylique

ester dipropylique d'acide 1,2,6-triméthyl-4-(4-bromophényl)-1,4-dihydropyridine-3,5-dicarboxylique

ester dibutylique d'acide 1,2,6-triméthyl-4-(4-bromophényl)-1,4-dihydropyridine-3,5-dicarboxylique

ester diéthylique d'acide 1-cyclopropyl-2,6-diméthyl-4-(3-trifluorométhylphényl)-1,4-dihydropyridine-3,5-dicarboxylique

ester diméthylique d'acide 1-éthyl-2,6-diméthyl-4-(4-trifluorométhylphényl)-1,4-dihydropyridine-3,5-dicarboxylique

ester diméthylique d'acide 1-cyclopropyl-2,6-diméthyl-4-(4-trifluorométhylphényl)-1,4-dihydropyridine-3,5-dicarboxylique

ester diéthylique d'acide 1-cyclopropyl-2,6-diméthyl-4-(4-trifluorométhylphényl)-1,4-dihydropyridine-3,5-dicarboxylique

ester diisopropylique d'acide 1,2,6-triméthyl-4-(4-trifluorométhylphényl)-1,4-dihydropyridine-3,5-dicarboxylique

ester diméthylique d'acide 1-cyclopropyl-2,6-diméthyl-4-(4-trifluorométhylphényl)-1,4-dihydropyridine-3,5-dicarboxylique

ester diéthylique d'acide 1-cyclopropyl-2,6-diméthyl-4-(4-trifluorométhoxyphényl)-1,4-dihydropyridine-3,5-dicarboxylique

ester d'isopropyle et de 2-méthoxyéthyle d'acide 1,2,6-triméthyl-4-(4-trifluorométhylphényl)-1,4-dihydropyridine-3,5-dicarboxylique

ester diéthylique d'acide 1,2,6-triméthyl-4-(4-trifluorométhylphényl)-1,4-dihydropyridine-3,5-dicarboxylique

ester de méthyle et d'éthyle d'acide 1-cyclopropyl-2,6-diméthyl-4-(4-trifluorométhylphényl)-1,4-dihydropyridine-3,5-dicarboxylique

ester de propyle et de 2-méthoxyéthyle d'acide 1-cyclopropyl-2,6-diméthyl-4-(4-trifluorométhylphényl)-1,4-dihydropyridine-3,5-dicarboxylique

ester d'isopropyle et de 2-méthoxyéthyle d'acide 1-cyclopropyl-2,6-diméthyl-4-(4-trifluorométhylphényl)-1,4-dihydropyridine-3,5-dicarboxylique

ester diéthylique d'acide 1-cyclopropyl-2,6-diméthyl-4-(4-fluorophényl)-1,4-dihydropyridine-3,5-dicarboxylique

ester diméthylique d'acide 1-cyclopropyl-2,6-diméthyl-4-(4-fluorophényl)-1,4-dihydropyridine-3,5-dicarboxylique

ester de propyle et de butyle d'acide 1-cyclopropyl-2,6-diméthyl-4-(4-trifluorophényl)-1,4-dihydropyridine-3,5-dicarboxylique

ester de butyle et de méthyle d'acide 1-cyclopropyl-2,6-diméthyl-4-(4-trifluorométhylphényl)-1,4-dihydropyridine-3,5-dicarboxylique

ester d'éthyle et de propyle d'acide 1-cyclopropyl-2,6-diméthyl-4-(4-trifluorométhylphényl)-1,4-dihydropyridine-3,5-dicarboxylique

ester de butyle et d'éthyle d'acide 1-cyclopropyl-2,6-diméthyl-4-(4-trifluorométhylphényl)-1,4-dihydropyridine-3,5-dicarboxylique

ester d'éthyle et d'isopropyle d'acide 1-cyclopropyl-2,6-diméthyl-4-(4-trifluorométhylphényl)-1,4-dihydropyridine-3,5-dicarboxylique,

caractérisé en ce que :

[A] on fait réagir des composés benzylidéniques de formule générale II

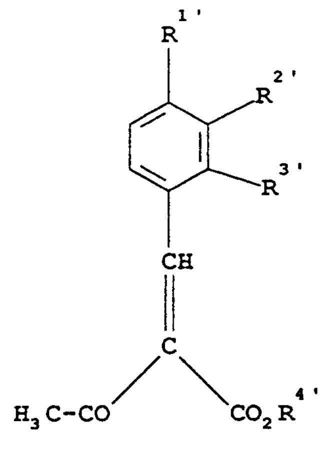

(II)

dans laquelle

$R^{1'}$, $R^{2'}$, $R^{3'}$ et $R^{4'}$ ont les limites de définition de chacun des composés nouveaux indiqués ci-dessus,

tout d'abord avec des esters d'acide $\beta$-aminocrotonique de formule générale (III)

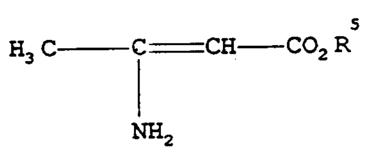

(III)

dans laquelle $R^{5'}$ a les limites de définition de chacun des composés nouveaux indiqués,

dans des solvants inertes et, dans une dernière étape, on alkyle la fonction -NH par un procédé classique, ou bien on fait réagir directement les composés de formule générale (II), le cas échéant en présence d'acides de Lewis tels que le tétrachlorure de titane, avec des composés de formule générale (IIIa)

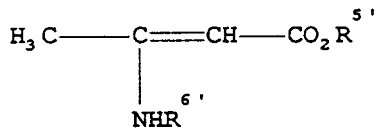 (IIIa)

dans laquelle

$R^{5'}$ et $R^{6'}$ ont les limites de définition de chacun des composés nouveaux indiqués ci-dessus, ou bien

[B] on fait réagir des aldéhydes de formule générale (IV)

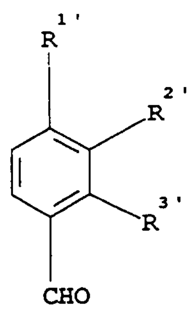 (IV)

dans laquelle

$R^{1'}$, $R^{2'}$, $R^{3'}$ ont la définition indiquée pour le procédé [A], tout d'abord avec des esters d'acides $\beta$-cétocarboxyliques de formules générales (V) et (Va)

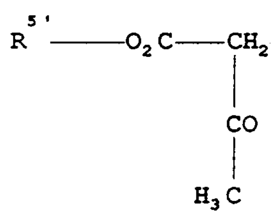 (V) et 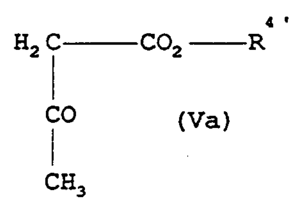 (Va)

dans lesquelles

$R^{4'}$ et $R^{5'}$ ont également la définition indiquée pour le procédé [A], puis on fait réagir le produit directement avec des amines ou les chlorhydrates d'amines correspondants de formule générale (VI)

$H_2N—R^{6'}$ (VI)

dans laquelle $R^{6'}$ a la définition indiquée pour le procédé [A] et on effectue tout d'abord une cyclisation par un procédé classique avec de l'ammoniac dans des solvants organiques éventuellement inertes et, dans une dernière étape, une alkylation par le procédé indiqué ci-dessus, et dans le cas des esters de pureté énantiomérique, on prépare tout d'abord les acides carboxyliques de pureté énantiomérique et on les estérifie par un procédé classique avec les alcools correspondants, en passant le cas échéant par un dérivé d'acide réactif.

**2.** Procédé de préparation de médicaments, caractérisé en ce qu'on fait prendre une forme administrable appropriée à au moins l'un des composés de dihydropyridine mentionnés dans la revendication 1, le cas échéant avec des substances auxiliaires et des supports classiques.